(19) Europäisches Patentamt
European Patent Office
Office européen des brevets

(11) EP 3 083 991 B1

(12) **EUROPEAN PATENT SPECIFICATION**

(45) Date of publication and mention of the grant of the patent:
**14.03.2018 Bulletin 2018/11**

(21) Application number: **14820805.1**

(22) Date of filing: **15.12.2014**

(51) Int Cl.:
***C12Q 1/68*** *(2018.01)*

(86) International application number:
**PCT/EP2014/077667**

(87) International publication number:
**WO 2015/091306 (25.06.2015 Gazette 2015/25)**

# (54) BIOMARKERS FOR HBV TREATMENT RESPONSE

BIOMARKER FÜR DAS ANSPRECHEN AUF EINE HBV-BEHANDLUNG

BIOMARQUEURS POUR LA RÉPONSE AU TRAITEMENT DU VIRUS DE L'HÉPATITE B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2013 EP 13197711**

(43) Date of publication of application:
**26.10.2016 Bulletin 2016/43**

(73) Proprietor: **F. Hoffmann-La Roche AG**
**4070 Basel (CH)**

(72) Inventors:
- **HE, Hua**
  **London W3 7BS (GB)**
- **WAT, Cynthia**
  **London NW6 1DZ (GB)**

(74) Representative: **Halbig, Dirk et al**
**F. Hoffmann-La Roche AG**
**Patent Department**
**Grenzacherstrasse 124**
**4070 Basel (CH)**

(56) References cited:
**WO-A1-97/13875** **WO-A1-2010/135649**
**WO-A2-2013/141705**

- **LOUIS JANSEN ET AL: "Genome wide association study of Chronic Hepatitis B patients identified a variation in the SLC16A9 gene which is associated with HBsAg loss after treatment with peginterferon and adefovi", HEPATOLOGY, vol. 56, no. suppl. 1, 1 October 2012 (2012-10-01), page 411A, XP055069962, USA ISSN: 0270-9139**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to methods that are useful for predicting the response of hepatitis B virus (HBV) infected patients to pharmacological treatment.

### Background of the invention

The hepatitis B virus (HBV) infects 350-400 million people worldwide; one million deaths resulting from cirrhosis, liver failure, and hepatocellular carcinoma due to the infection are recorded annually. The infecting agent, hepatitis B virus (HBV), is a DNA virus which can be transmitted percutaneously, sexually, and perinatally. The prevalence of infection in Asia (≥ 8 %) is substantially higher than in Europe and North America (<2%) (Dienstag J.L., Hepatitis B Virus Infection., N. Engl. J. Med. 2008; 359: 1486-1500). The incidence of HBV acquired perinatally from an infected mother is much higher in Asia, leading to chronic infection in >90% of those exposed (WHO Fact Sheet No 204; revised August 2008). Additionally, 25% of adults who become chronically infected during childhood die from HBV-related liver cancer or cirrhosis (WHO Fact Sheet No 204; revised August 2008). Interferon alpha (IFN$\alpha$) is a potent activator of anti-viral pathways and additionally mediates numerous immuno-regulatory functions (Muller U., Steinhoff U., Reis L.F. et al., Functional role of type I and type II interferons in antiviral defense, Science 1994; 264: 1918-21).

The efficacy of PEGASYS® (Pegylated IFN alfa 2a 40KD, Peg-IFN) at a dose of 180$\mu$g/week in the treatment of HBV was demonstrated in two large-scale pivotal studies. One study was in HBeAg-negative patients (WV16241) and the other in HBeAg-positive patients (WV16240).

WV16241 was conducted between June 2001 and August 2003; 552 HBeAg-negative CHB patients were randomized to one of three treatment arms: PEG-IFN monotherapy, PEG-IFN plus lamivudine or lamivudine alone for 48 weeks. Virologic response (defined as HBV DNA <20,000 copies/mL) assessed 24 weeks after treatment cessation was comparable in the groups that received PEG-IFN (43% and 44%) and both arms were superior to the lamivudine group (29%) (Marcellin P., Lau G.K., Bonino F. et al., Peginterferon alfa-2a alone, lamivudine alone, and the two in combination in patients with HBeAg-negative chronic hepatitis B, N. Engl. J. Med. 2004; 351: 1206-17).

Study WV16240 was conducted between January 2002 and January 2004. In this study, 814 HBeAg-positive CHB patients were randomized to the same treatment arms as in WV16241, i.e. PEG-IFN monotherapy, PEG-IFN plus lamivudine or lamivudine alone for 48 weeks. Responses assessed 24 weeks after treatment cessation showed a 32% rate of HBeAg seroconversion in the PEG-IFN monotherapy group compared to 27% and 19% with PEG-IFN + lamivudine and lamivudine monotherapy respectively (Lau G.K., Piratvisuth T,. Luo K.X. et al., Peginterferon Alfa-2a, Lamivudine, and the Combination for HBeAg-Positive Chronic Hepatitis B, N. Engl. J. Med. 2005; 352: 2682-95). Metaanalysis of controlled HBV clinical studies has demonstrated that PEG-IFN-containing treatment facilitated significant HBsAg clearance or seroconversion in CHB patients over a lamivudine regimen (Li W.C., Wang M.R., Kong L.B. et al., Peginterferon alpha-based therapy for chronic hepatitis B focusing on HBsAg clearance or seroconversion: a meta-analysis of controlled clinical trials, BMC Infect. Dis. 2011; 11: 165-177).

More recently, the Neptune study (WV 19432) was conducted between May 2007 and April 2010 and compared PEG-IFN administered as either 90 or 180 $\mu$g/week administered over either 24 or 48 weeks in HBeAg-positive patients (Liaw Y.F., Jia J.D., Chan H.L. et al., Shorter durations and lower doses of peginterferon alfa-2a are associated with inferior hepatitis B e antigen seroconversion rates in hepatitis B virus genotypes B or C, Hepatology 2011; 54: 1591-9). Efficacy was determined at 24 weeks following the end of treatment. This study, demonstrated that both the lower dose and shorter durations of treatment were inferior to the approved dose and duration previously used in the WV16240 study, thus confirming that the approved treatment regimen of i.e. 180$\mu$g/week for 48 weeks is the most beneficial for patients with HBeAg-positive CHB.

However, despite the fact that PEG-IFN has been successfully used in the treatment of CHB, little is known of the impact of host factors (genetic and non-genetic) and viral factors on treatment response.

Although viral and environmental factors play important roles in HBV pathogenesis, genetic influence is clearly present. While small genetic studies have suggested the possible implications of host immune/inflammation factors (e.g. HLA, cytokine, inhibitory molecule) in the outcomes of HBV infection, a genome-wide association study (GWAS) clearly demonstrated that 11 single nucleotide polymorphisms (SNPs) across the human leukocyte antigen (HLA)-DP gene region are significantly associated with the development of persistent chronic hepatitis B virus carriers in the Japanese and Thai HBV cohorts (Kamatani Y., Wattanapokayakit S., Ochi H. et al., A genome-wide association study identifies variants in the HLA-DP locus associated with chronic hepatitis B in Asians. Nat. Genet. 2009; 41: 591-595). Subsequently this finding was also confirmed in a separate Chinese cohort study using a TaqMan based genotyping assay (Guo X., Zhang Y., Li J. et al., Strong influence of human leukocyte antigen (HLA)-DPgene variants on development of persistent chronic hepatitis B virus carriers in the Han Chinese population, Hepatology 2011; 53: 422-8). Furthermore, a separate GWAS and replication analysis concluded similar results that there is significant association between the HLA-DP locus and the protective effects against persistent HBV infection in Japanese and Korean populations (Nishida N., Sawai H.,

Matsuura K. et al., Genome-wide association study confirming association of HLA-DP with protection against chronic hepatitis B and viral clearance in Japanese and Korean. PLos One 2012; 7: e39175). Finally, two additional SNPs (rs2856718 and rs7453920) within the HLA-DQ locus were found to have an independent effect of HLA-DQ variants on CHB susceptibility (Mbarek H., Ochi H., Urabe Y. et al., A genome-wide association study of chronic hepatitis B identified novel risk locus in a Japanese population, Hum. Mol. Genet. 2011; 20: 3884-92). Taken together, robust genetic evidence suggests that in the Asian population, polymorphic variations at the HLA region contribute significantly to the progression of chronic hepatitis B following acute infection in Asian populations.

Meta-analysis of controlled HBV clinical trials has demonstrated that conventional IFN alfa- or pegylated IFN alfa (2a or 2b)-containing treatment facilitated significant HBsAg clearance or seroconversion in CHB patients over lamivudine regimens (Li W.C., Wang M.R., Kong L.B. et al., Peginterferon alpha-based therapy for chronic hepatitis B focusing on HBsAg clearance or seroconversion: a meta-analysis of controlled clinical trials, BMC Infect. Dis. 2011; 11: 165-177). However, despite the fact that Peg-IFN has been successfully used in the treatment of CHB, little is known regarding the relationship between treatment response and the impact of host factors at the level of single nucleotide polymorphisms (SNPs). Pegylated interferon alfa, in combination with ribavirin (RBV) has been successfully used in the treatment of chronic hepatitis C virus (HCV) infection. A major scientific finding in how HCV patients respond to Peg-IFN/RBV treatment is that via genome-wide association studies (GWAS), genetic polymorphisms around the gene IL28B on chromosome 19 are strongly associated with treatment outcome (Ge D., Fellay J., Thompson A.J. et al., Genetic variation in IL28B predicts hepatitis C treatment-induced viral clearance, Nature 2009; 461: 399-401; Tanaka Y., Nishida N., Sugiyama M. et al., Genome-wide association of IL28B with response to pegylated interferon-alpha and ribavirin therapy for chronic hepatitis C, Nat. Genet. 2009; 41: 1105-9; Suppiah V., Moldovan M., Ahlenstiel G. et al., IL28B is associated with response tochronic hepatitis C interferon-alpha and ribavirin therapy, Nat. Genet. 2009; 41: 1100-4). IL28B encoded protein is a type III IFN (IFN-$\lambda$3) and forms a cytokine gene cluster with IL28A and IL29 at the same chromosomal region. IL28B can be induced by viral infection and has antiviral activity. However, in CHB patients treated with Peg-IFN, there are limited and somewhat conflicting data on the association of specific SNPs (e.g. rs12989760, rs8099917, rs12980275) around IL28B region with treatment responses (Lampertico P., Vigano M., Cheroni C. et al., Genetic variation in IL28B polymorphism may predict HBsAg clearance in genotype D, HBeAg negative patients treated with interferon alfa, AASLD 2010; Mangia A., Santoro R., Mottola et al., Lack of association between IL28B variants and HBsAg clearance after interferon treatment, EASL 2011; de Niet A., Takkenberg R.B., Benayed R. et al., Genetic variation in IL28B and treatment outcome in HBeAg-positive and -negative chronic hepatitis B patients treated with Peg interferon alfa-2a and adefovir, Scand. J. Gastroenterol. 2012, 47: 475-81; Sonneveld M.J., Wong V.W., Woltman A.M. et al., Polymorphisms near IL28B and serologic response to peginterferon in HBeAg-positive patients with chronic hepatitis B, Gastroenterology 2012; 142: 513-520).

IL28B genotype predicts response to pegylated-interferon (peg-IFN)-based therapy in chronic hepatitis C. Holmes et al. investigated whether IL28B genotype is associated with peg-IFN treatment outcomes in a predominantly Asian CHB cohort. IL28B genotype was determined for 96 patients (Holmes et al., IL28B genotype is not useful for predicting treatment outcome in Asian chronic hepatitis B patients treated with pegylated interferon-alpha, J. Gastroenterol. Hepatol., 2013, 28(5): 861-6). 88% were Asian, 62% were HBeAg-positive and 13% were METAVIR stage F3-4. Median follow-up time was 39.3 months. The majority of patients carried the CC IL28B genotype (84%). IL28B genotype did not differ according to HBeAg status. The primary endpoints were achieved in 27% of HBeAg-positive and 61% of HBeAg-negative patients. There was no association between IL28B genotype and the primary endpoint in either group. Furthermore, there was no difference in HBeAg loss alone, HBsAg loss, ALT normalisation or on-treatment HBV DNA levels according to IL28B genotype.

With whole blood sample collection in CHB patients who have been treated with Peg-IFN and have definite clinical outcomes, it is well justified that mechanistically understanding how host genetic factors affect treatment response and HBV disease biology will be tremendously beneficial to the future clinical practice of identifying patients who are likely to respond to Peg-IFN treatment and to the development of new HBV medicines.

## Summary of the invention

The present invention provides for methods for identifying patients who will respond to an anti-HBV treatment with anti-HBV agents, such as an interferon.

One embodiment of the invention provides methods of identifying a patient who may benefit from treatment with an anti-HBV therapy comprising an interferon, the methods comprising: determining the presence of a single nucleotide polymorphism in gene *SON* on chromosome 21 in a sample obtained from the patient, wherein the presence of at least one G allele at rs13047599 indicates that the patient may benefit from the treatment with the anti-HBV treatment. Another embodiment of the present invention provides methods for predicting HBeAg seroconversion and HBV DNA <2000 IU/ml at >=24-week follow-up of treatment of an HBe-positive patient infected with HBV to interferon treatment comprising: (i) detecting the presence of a single nucleotide polymorphism in gene *SON* on chromosome 21 in a sample

obtained from said patient, and (ii) determining that said patient has a high response rate to interferon treatment measured as HBeAg seroconversion and HBV DNA <2000 IU/ml at >=24-week follow-up of treatment if at least one G allele at rs13047599 is present.

In some embodiments, the interferon is selected from the group of peginterferon alfa-2a, peginterferon alfa-2b, interferon alfa-2a and interferon alfa-2b.

In some embodiments, the interferon is a peginterferon alfa-2a conjugate having the formula:

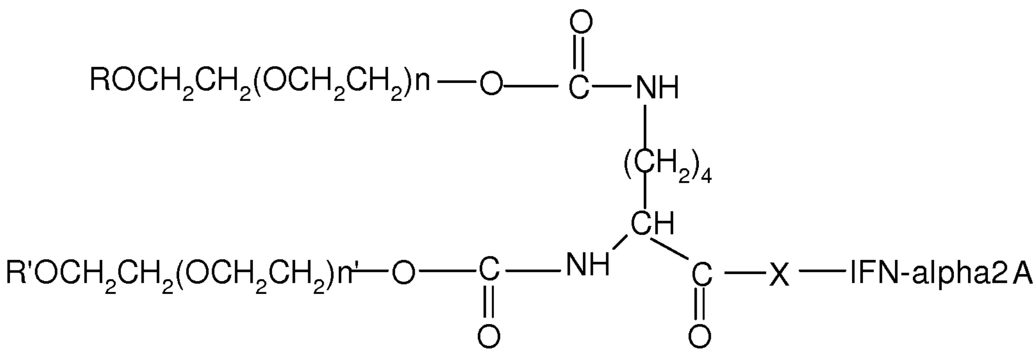

wherein R and R' are methyl, X is NH, and n and n' are individually or both either 420 or 520.

Brief description of the drawings

**Figure 1:** Bar chart of the number of markers by chromosome in the *GWAS Marker Set.* An additional 1095 markers, were not plotted due to unknown genomic location.

**Figure 2:** Scree plot for ancestry analysis. It is clear that the majority of information (highest eigenvalues) are obtained from the first two principal components of ancestry, with little gain in information after the fifth principal component.

**Figure 3:** The first three principal components of ancestry for HapMap individuals only. Population codes are as listed in Table 2. Figure 3 shows the results of PCA for the HapMap reference data only. Five clusters are visible, corresponding to five major bio-geographic ancestral origins. Reading clockwise from top left, they are: African origin (blue/ orange/ pink/ maroon), South Asian Origin (grey), Southeast Asian (yellow/ blue/ green), Mexican (dark green) and Northern and Western European (blue/ red).

**Figure 4:** The first three principal components of ancestry for HapMap individuals; coloured according to population group (Table 2). Overlaid are patients who self-report as 'Oriental' (black crosses) or another racial group (grey crosses).first three principal components of ancestry for HapMap individuals only. Population codes are as listed in Table 2.

**Figure 5 :** Manhattan Plots for Endpoint 1

**Figure 6 :** Manhattan Plots for Endpoint 2

**Figure 7 :** Manhattan Plots for Endpoint 4

**Figure 8:** Bar plot of rate of response (Endpoint 1) for rs13047599 under the dominant model.

**Figure 9:** Bar plot of rate of response (Endpoint 4) for rs 12000 under the dominant model

**Figure 10:** Bar plot of rate of response (Endpoint 4) for rs 1913228 under the dominant model

**Figure 11:** Bar plot of rate of response (Endpoint 4) for rs 12636581 under the dominant model

## Detailed description of the invention

### Definitions

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The terms "sample" or "biological sample" refers to a sample of tissue or fluid isolated from an individual, including, but not limited to, for example, tissue biopsy, plasma, serum, whole blood, spinal fluid, lymph fluid, the external sections of the skin, respiratory, intestinal and genitourinary tracts, tears, saliva, milk, blood cells, tumors, organs. Also included are samples of in vitro cell culture constituents (including, but not limited to, conditioned medium resulting from the growth of cells in culture medium, putatively virally infected cells, recombinant cells, and cell components).

The terms "interferon" and "interferon-alpha" are used herein interchangeably and refer to the family of highly homologous species-specific proteins that inhibit viral replication and cellular proliferation and modulate immune response. Typical suitable interferons include, but are not limited to, recombinant interferon alpha-2b such as Intron® A interferon available from Schering Corporation, Kenilworth, N.J., recombinant interferon alpha-2a such as Roferon®-A interferon available from Hoffmann-La Roche, Nutley, N.J., recombinant interferon alpha-2C such as Berofor® alpha 2 interferon available from Boehringer Ingelheim Pharmaceutical, Inc., Ridgefield, Conn., interferon alpha-n1, a purified blend of natural alpha interferons such as Sumiferon® available from Sumitomo, Japan or as Wellferon® interferon alpha-n1 (INS) available from the Glaxo-Wellcome Ltd., London, Great Britain, or a consensus alpha interferon such as those described in U.S. Pat. Nos. 4,897,471 and 4,695,623 (especially Examples 7, 8 or 9 thereof) and the specific product available from Amgen, Inc., Newbury Park, Calif., or interferon alpha-n3 a mixture of natural alpha interferons made by Interferon Sciences and available from the Purdue Frederick Co., Norwalk, Conn., under the Alferon Tradename. The use of interferon alpha-2a or alpha-2b is preferred. Interferons can include pegylated interferons as defined below.

The terms "pegylated interferon", "pegylated interferon alpha" and "peginterferon" are used herein interchangeably and means polyethylene glycol modified conjugates of interferon alpha, preferably interferon alfa-2a and alfa-2b. Typical suitable pegylated interferon alpha include, but are not limited to, Pegasys® and Peg-Intron®.

As used herein, the terms "allele" and "allelic variant" refer to alternative forms of a gene including introns, exons, intron/exon junctions and 3' and/or 5' untranslated regions that are associated with a gene or portions thereof. Generally, alleles occupy the same locus or position on homologous chromosomes. When a subject has two identical alleles of a gene, the subject is said to be homozygous for the gene or allele. When a subject has two different alleles of a gene, the subject is said to be heterozygous for the gene. Alleles of a specific gene can differ from each other in a single nucleotide, or several nucleotides, and can include substitutions, deletions, and insertions of nucleotides.

As used herein, the term "polymorphism" refers to the coexistence of more than one form of a nucleic acid, including exons and introns, or portion (e.g., allelic variant) thereof. A portion of a gene of which there are at least two different forms, i.e., two different nucleotide sequences, is referred to as a polymorphic region of a gene. A polymorphic region can be a single nucleotide, i.e. "single nucleotide polymorphism" or "SNP", the identity of which differs in different alleles. A polymorphic region can also be several nucleotides long.

Numerous methods for the detection of polymorphisms are known and may be used in conjunction with the present invention. Generally, these include the identification of one or more mutations in the underlying nucleic acid sequence either directly (e.g., in situ hybridization) or indirectly (identifying changes to a secondary molecule, e.g., protein sequence or protein binding).

One well-known method for detecting polymorphisms is allele specific hybridization using probes overlapping the mutation or polymorphic site and having about 5, 10, 20, 25, or 30 nucleotides around the mutation or polymorphic region. For use in a kit, e.g., several probes capable of hybridizing specifically to allelic variants, such as single nucleotide polymorphisms, are provided for the user or even attached to a solid phase support, e.g., a bead or chip.

The single nucleotide polymorphism, "rs 13047599" refers to a SNP identified by its accession number in the database of SNPs (dbSNP, www.ncbi.nlm.nih.gov/SNP/) and is located on human chromosome 21 in the *SON DNA binding protein* gene.

### Abbreviations

| | |
|---|---|
| AIC | Akaike Information Criterion |
| ALT | Alanine aminotransferase |
| Anti-HBs | Antibody to hepatitis B surface antigen |

(continued)

| | |
|---|---|
| DNA | Deoxyribonucleic acid |
| GWAS | Genome-wide Association Study |
| HAV | Hepatitis A Virus |
| HBe | Hepatitis B 'e' Antigen |
| HBeAg | Hepatitis B 'e' Antigen |
| HBV | Hepatitis B Virus |
| HCV | Hepatitis C Virus |
| HIV | Human Immunodeficiency Virus |
| HLA | Human Leucocyte Antigen |
| HWE | Hardy-Weinberg Equilibrium |
| IU/ml | International units per milliliter |
| PCA | Principal Components Analysis |
| PEGASYS | Pegylated Interferon alpha 2a 40KD; Peg-IFN |
| Peg-IFN | Pegylated Interferon alpha 2a 40KD; PEGASYS |
| QC | Quality Checks |
| qHBsAg | Quantitative Hepatitis B Surface Antigen |
| S-loss | Surface Antigen Loss |
| SNP | Single Nucleotide Polymorphism |
| SPC | Summary of Product Characteristics |
| TLR | Toll-like Receptor |
| Tx | Treatment |
| Vs. | Versus |

## Examples

### Objectives and Endpoints

The objective was to determine genetic variants associated with response to treatment with PEGASYS-containing regimen in patients with Chronic Hepatitis B.

The following endpoints, by patient group, were considered.

### HBe-positive patients:

1. HBeAg seroconversion at >=24-week follow-up (responders vs. non-responders)
2. HBeAg seroconversion plus HBV DNA<2000 IU/ml at >=24-week follow-up (responders vs. non-responders)
3. Loss of HBsAg or seroconversion at >=24- week follow-up (responders vs. non-responders)

The above list of endpoints shall hereafter be referred to as Endpoints 1-3 respectively.

### HBe-negative patients:

4. HBV DNA<2000 IU/ml at >=24-week follow-up (responders vs. non-responders)

The latter shall be referred to as Endpoint 4.

Endpoints 3 and 4 were analyzed also in the combined set of HBe-positive and HBe-negative patients.

For all endpoints and all markers, the null hypothesis of no association, between the genotype and the endpoint, was tested against the two-sided alternative that association exists.

Study Design

A cumulative meta-analysis, of data from company-sponsored clinical trials, and data from patients in General Practice care, is in progress. The combined data will, at the final analysis, comprise up to 1500 patients who have been treated with Pegasys for at least 24 weeks, with or without a nucleotide/ nucleoside analogue, and with 24 weeks of follow-up data available.

The following trials/ patient sources were considered for inclusion:

- RGT (ML22266)
- S-Collate (MV22009)
- SoN (MV22430)
- Switch (ML22265)
- Combo
- New Switch (ML27928)
- NEED
- Italian cohort of PEG.Be.Liver
- Professor Teerha (Thailand): clinical practice patients and some legacy Ph3 patients
- Professor Hongfei Zhang (Beijing, China): clinical practice patients and some legacy Ph3 patients
- Professor Yao Xie (Beijing, China): clinical practice patients
- Professor Xin Yue Chen (Beijing, China): clinical practice patients

Adult patients with chronic hepatitis B (male or female patients ≥18 years of age) must meet the following criteria for study entry:

- Previously enrolled in a Roche study and treated for chronic hepatitis B for at least 24 weeks with Peg-IFN ± nucleoside analogue (lamivudine or entacavir) or Peg-IFN ± nucleotide analogue (adefovir) with ≥24-week post-treatment follow-up or;
- Treated in general practice for chronic hepatitis B with Peg-IFN according to standard of care and in line with the current summary of product characteristics (SPC) / local labeling who have no contra-indication to Peg-IFN therapy as per the local label and have been treated with Peg-IFN for at least 24 weeks and have ≥24-week post-treatment response available at the time of blood collection.
- Patients are not infected with HAV, HCV, or HIV
- Patients should have the following medical record available (either from historical/ongoing study databases or from medical practice notes):
    - Demographics (e.g. age, gender, ethnic origin)
    - Pre-therapy HBeAg status, known or unknown HBV genotype
    - Quantitative HBV DNA by PCR Test in IU/ml over time (e.g. baseline, on-treatment: 12- and 24-week, post-treatment: 24-week)
    - Quantitative HBsAg test (if not available, qualitative HBsAg test) and anti-HBs over time (e.g. baseline, on-treatment: 12- and 24-week, post-treatment: 24-week)
    - Serum ALT over time (e.g. baseline, on-treatment: 12- and 24-week, post-treatment: 24-week)

It is noted that all patients will have received active regimen.

Analysis Populations

The majority of patients will be from China. For the purposes of statistical analysis, four analysis populations were defined as follows.

- *PGx-FAS* is all patients with at least one genotype
- *PGx-GT* is the subset of PGx-*FAS* whose genetic data passes quality checks
- *PGx-CN* is the subset of *PGx-GT* who share a common genetic background in the sense that they cluster with CHB

and CHD reference subjects from HapMap version3 (see below)

- *PGx-non-CN* is the remainder of *PGx-GT* who do not fall within *PGx-CN*

Additional suffices are appended as *HBePos* or *HBeNeg* for the HBe-Positive and HBe-Negative subsets respectively, and as *interim1,... interim3,* and *final,* according to the stage of the analysis.

Genetic Markers

The GWAS marker panel was the Illumina OmniExpress Exome microarray (www.illumina.com), consisting of greater than 750,000 SNP markers and greater than 250,000 exonic markers. The group of markers which passed quality checks are referred to as the *GWAS Marker Set.*

General Considerations for Data Analysis

The GWAS is hypothesis-free. Markers with unadjusted $p<5x10^{-8}$ were considered to be genome-wide significant. In the interests of statistical power, no adjustment was made for multiple endpoints or multiple rounds of analysis.

Results for First Interim Analysis

The following paragraphs describe the results arising from the first interim analysis. Similar analyses will be conducted for up to three further batches of accumulating data, with patient sets labeled appropriately with suffices: *interim2, interim3,* and *final.*

Description of the Data

Clinical data was received in the form of a comma-delimited flat file entitled *demoext.csv.* The file had 20 columns and 218 rows including a header line, with one row per patient.

Genetic data was received in the form of four Illumina-formatted files entitled, 24-*luoshi_FinalReport.txt, 25_FinalReport.txt, 32_FinalReport.txt* and *56_FinalReport.txt.* The combination of these files contained genotypes for 137 patients, and 951,117 SNPs.

Table 1 below shows the baseline and demographic characteristics for the 137 patients with at least one genotype. All patients were studied under protocol MV22430. The subject set is *PGx-FAS-interiml .*

**Table 1: Baseline and Demographic Characteristics for *PGx-FAS-interim1***

| Variable | Category | Statistics | Result |
|---|---|---|---|
| Count (n) | | | 137 |
| Sex | Male | n (%) | 88 (64%) |
| | Female | n (%) | 49 (36%) |
| Age (yr) | | Mean (SE) | 32.25 (0.848) |
| Race | Oriental | n (%) | 119 (87%) |
| | Caucasian | n (%) | 7 (5%) |
| | Other* | n (%) | 11 (8%) |
| Height (cm) | | Mean (SE) | 168.26 (0.766) |
| Weight (kg) | | Mean (SE) | 67.74 (1.43) |
| BMI (kg/m^2) | | Mean (SE) | 23.78 (0.416) |
| Baseline ALT (U/L) | | Median (IQR) | 123 (119) |
| *Self reported races were as follows: Pacific Islander; Maori; Indian; Burmese; Black | | | |

Quality Checks by Patient

The following criteria were assessed, on the basis of unfiltered GWAS data, in all 137 patients of any self-reported race (*PGx-FAS-interim1)*.

- <5% missing genotype data
- <30% heterozygosity genome-wide
- <30% genotype-concordance with another sample
- Reported sex consistent with X-chromosome data

All patients had <5% missing genotypes, genome-wide heterozygosity < 30% and X-chromosome data consistent with self-reported sex. One pair of first-degree relatives however was detected. The patient identifiers (ANONID) were 8734 (female Caucasian, aged 55yr) and 8760 (male Caucasian, aged 25 yr). Of the two, patient 8734 had slightly more missing data (0.2% vs. 0.1%) and so will be excluded from further analysis.

The remaining 136 patients were incorporated into the *PGx-GT-interim1 Set.*

Quality Checks by Marker

Markers derived for GWAS and candidate gene study were assessed for missing data. A total of 1712 markers (<0.2% of the total) had greater than 5% missing data and were excluded from further analysis. The *GWAS Marker Set* therefore consists of 949,405 markers. Figure 1 shows the number of markers by chromosome in the *GWAS Marker Set.* As expected across the autosomes, the number of markers varies approximately in line with chromosomal size.

Multivariate Analysis of Ancestry

Principal Components Analysis (PCA) is a technique for reducing the dimensionality of a data set. It linearly transforms a set of variables into a smaller set of uncorrelated variables representing most of the information in the original set (Dunteman, 1989). In the current study, the marker variables were transformed into principal components which were compared to self-reported ethnic groupings. The objective is, in preparation for association testing, to determine clusters of individuals who share a homogeneous genetic background.

A suitable set of GWAS markers for ancestry analysis was obtained, using *PGx-GT-interim1,* as follows. Markers were excluded if they had frequency less than 5% or if they corresponded to regions with known high Linkage disequilibrium (LD) or inversion (Chr5, 44-51.5Mb; Chr6, 24-36Mb; Chr8, 8-12Mb; Chr11, 42-58Mb; Chr17, 40-43Mb). In order to facilitate merging, markers encoding complementary base-changes were also removed. The remaining markers were thinned such that all SNPs within a window of size 1000 had $r^2$<0.25.

HapMap version 3 data was downloaded for the resultant marker set (The International HapMap Consortium, 2003; 2005; 2007). Table 2 shows the composition of the HapMap subjects, who were used as reference sets against which data from *PGx-GT-interim1* was compared. HapMap data were merged with *PGx-GT-interim1* data, taking care to resolve any strand differences between the two sources. Any marker not available for HapMap subjects was excluded from the merged file.

PCA was applied using 134,575 markers, selected as described above, and genotyped across 136 study individuals and 988 HapMap reference individuals.

**Table 2: Details of the HapMap version 3 reference subjects**

| Code | Description | Count |
|---|---|---|
| MKK | Maasai in Kinyawa, Kenya | 143 |
| LWK | Luhya in Webuye, Kenya | 90 |
| YRI | Yoruba in Ibadan, Nigeria | 113 |
| ASW | African ancestry in Southwest USA | 49 |
| CEU | Utah residents with Northern and Western European ancestry from the CEPH collection | 112 |
| TSI | Tuscans in Italy | 88 |
| MEX | Mexican ancestry in Los Angeles, California | 50 |
| GIH | Gujarati Indians in Houston, Texas | 88 |
| JPT | Japanese in Tokyo, Japan | 86 |
| CHD | Chinese in Metropolitan Denver, Colorado | 85 |

(continued)

| Code | Description | Count |
|---|---|---|
| CHB | Han Chinese in Bejing, China | 84 |
| | **TOTAL** | 988 |

Figure 4 shows the same data with study participants overlaid. Patients self-reporting as 'Oriental' are given by black crosses; patients self-reporting as another racial group are given by grey crosses. Two observations are of note. Firstly, it can be clearly seen that while those patients self-reporting as 'Oriental' cluster with, or close to, the Chinese and Japanese HapMap reference individuals, they form a much wider cluster. As such, the study participants represent a genetically more diverse group of individuals than the reference set. The study participants are likely to have been drawn from different countries in South-East Asia. Secondly, within the cluster of black crosses, some grey crosses are observed - these represent individuals who did not self-report as 'Oriental', but whose genetic background is indistinguishable from that of members of the 'Oriental' group.

For the purposes of genetic analysis, *PGx-CN-interim1* was made up of the 128 patients falling within the boundaries of the self-reported 'Oriental' cluster. Eight patients, whose plotted ancestry clearly departed from that cluster, made up *PGx-non-CN-interim1* : they self-reported as Caucasian (n=6), Maori (*n*=1) and Indian (*n*=1).7.

Assessment of Covariates

In order to determine the covariates for the forthcoming genome-wide association analysis, a series of variables were tested for association with each endpoint, using backwards stepwise regression. In accordance with the planned analysis, the subject set for Endpoints 1-3was PGx-GT-HBePos-Interim1 (n=134); the subject set for Endpoint 4 was all members of PGx-GT-Interim1 (n=136). Backwards steps were taken on the basis of the Akaike Information Criterion (AIC).

The covariates in the full model were as follows: Age, Sex, Baseline HBV DNA, Baseline ALT, HBV genotype, and Concomitant use of nucleotide/ nucleoside analogues. Baseline HBV and Baseline ALT were both log-transformed in order to improve symmetry. Due to the fact that almost all patients shared a homogeneous genetic background, principal components of ancestry were not included in the backwards stepwise regression. Tables 3-5 show the covariates selected for the various endpoints.

It can be seen that baseline HBV DNA and Baseline ALT were selected in all five instances; Concomitant nucleotide/ nucleoside analogues were selected in three of the five; HBV Genotype was selected for Endpoint 4, although the coding of that variable (with three low-frequency categories) meant that the individual effect sizes were not well-estimated.

**Table 3: Covariates selected by backwards stepwise regression for Endpoint 1**

| Variable | Odds Ratio (95% CI) | p-value |
|---|---|---|
| (Intercept) | 0.23 (0.00-12.94) | 0.4729 |
| Log(HBV DNA) | 0.55 (0.37-0.83) | 0.0037 |
| Log(ALT) | 3.03 (1.53-6.02) | 0.0015 |
| Concomitant nucleotide/ nucleoside analogues | 0.35 (0.14-0.86) | 0.0220 |

**Table 4: Covariates selected by backwards stepwise regression for Endpoint 2**

| Variable | Odds Ratio (95% CI) | p-value |
|---|---|---|
| (Intercept) | 0.04 (0.00-3.91) | 0.1673 |
| Log(HBV DNA) | 0.49 (0.30-0.79) | 0.0035 |
| Log(ALT) | 4.27 (1.85-9.86) | 0.0007 |
| Concomitant nucleotide/ nucleoside analogues | 0.32 (0.09-1.12) | 0.0736 |

**Table 5: Covariates selected by backwards stepwise regression for Endpoint 4**

| Variable | Odds Ratio (95% CI) | p-value |
|---|---|---|
| (Intercept) | 0.17 (0.00-10.28) | 0.3980 |
| Sex (Male) | 0.39 (0.15,1.04) | 0.0593 |
| Log(HBV DNA) | 0.44 (0.28,0.68) | 0.0002 |
| Log(ALT) | 3.75 (1.74,8.05) | 0.0007 |
| Genotype A | NA | 0.2292 |
| Genotype B | 4.18 (1.49,11.73) | 0.0066 |
| Genotype D | 0.70 (0.07,7.31) | 0.7618 |
| Genotype Mixed | NA | 0.0698 |

Single-Point Association Analysis

Methods

Due to the modest sample size (*n*=136) of the first interim analysis, markers were excluded from single-point association analysis if they had frequency less than 5%. The remaining 571832 markers were coded in two ways as follows. Firstly they were coded according to an additive model, given by the count of the number of minor alleles. Secondly they were coded according to a dominant model of inheritance, based upon carriage of the minor allele.

Association analysis was conducted for two patient sets and five endpoints, under two modes of inheritance.

The following model was fitted using multivariate logistic regression:

$$\text{Endpoint}=\text{Intercept} + [\text{Covariates}]+ \text{Marker}$$

Covariates were applied as selected above (Section 7.5). Despite the original intent, no correction for principal components of ancestry was applied in the analyses of *PGx-GT,* due to problems of over-fitting in this mainly homogeneous set. Adjustments for principal components of ancestry will be attempted in future interim analyses. An adjustment for study was also not applied because all of the patients in the current interim analysis are drawn from the same protocol.

The significance of each marker was determined using a t-test. The genomic control lambda was calculated for each GWAS analysis and QQ-plots were examined, but no clear evidence of test-statistic inflation was found (Devlin and Roeder 1999).

All markers were tested, using a chi-square test, for departure from Hardy-Weinberg Equilibrium (HWE). The calculation was performed for patients in the *PGx-CN-interim1 Set* and the results were used to assist in the interpretation of association analysis output.

Bar plots were produced for markers of interest, to show the rate of response by genotype.

Results

Figures 5-7 are the Manhattan Plots, by Endpoint and Tables 6-17 list association results with $p<10^{-4}$.

**Table 6 Association Results with $p<10^{-4}$ for Endpoint 1, *PGx-CN-HBePos,* additive model**

| Chr | SNP | BP | HWE(p) | MAF | Beta | p-value | Variant | Gene |
|---|---|---|---|---|---|---|---|---|
| 7 | rs16878220 | 14557271 | 1.0000 | 0.2930 | 5.23 | 4.20E-05 | INTRONIC | DGKB |
| 7 | rs10257158 | 14558492 | 1.0000 | 0.2930 | 5.90 | 1.92E-05 | INTRONIC | DGKB |
| 7 | rs16878221 | 14583406 | 0.6738 | 0.3008 | 6.46 | 1.62E-05 | INTRONIC | DGKB |
| 9 | rs3750551 | 71051949 | 0.5431 | 0.3164 | 5.14 | 7.10E-05 | INTRONIC | TJP2 |
| 17 | rs17689366 | 10118314 | 1.0000 | 0.1211 | 10.01 | 3.30E-05 | INTERGENIC | NA |
| 17 | rs17762691 | 10119173 | 1.0000 | 0.1250 | 9.21 | 4.52E-05 | INTERGENIC | NA |

(continued)

| Chr | SNP | BP | HWE(p) | MAF | Beta | p-value | Variant | Gene |
|---|---|---|---|---|---|---|---|---|
| 23 | rs1037218 | NA | 0.7654 | 0.4624 | 6.90 | 5.33E-05 | NA | NA |
| 23 | rs1026101 | NA | 1.0000 | 0.4451 | 6.24 | 8.72E-05 | NA | NA |
| 23 | rs5915908 | NA | 0.7654 | 0.4682 | 6.82 | 6.29E-05 | NA | NA |

**Table 7 Association Results with $p<10^{-4}$ for Endpoint 1), *PGx-GT-HBePos,* additive model**

| Chr | SNP | BP | HWE (p) | MAF | Beta | p-value | Variant | Gene |
|---|---|---|---|---|---|---|---|---|
| 5 | rs814586 | 10584998 | 0.578 | 0.3828 | 5.14 | 5.25E-05 | INTERGENIC | NA |
| 7 | rs16878220 | 14557271 | 1.0000 | 0.2930 | 5.00 | 3.61E-05 | INTRONIC | DGKB |
| 7 | rs10257158 | 14558492 | 1.0000 | 0.2930 | 5.59 | 1.61E-05 | INTRONIC | DGKB |
| 7 | rs16878221 | 14583406 | 0.6738 | 0.3008 | 5.67 | 2.01E-05 | INTRONIC | DGKB |
| 11 | rs2553825 | 35055962 | 0.7952 | 0.2109 | 4.87 | 9.56E-05 | INTERGENIC | NA |
| 17 | rs17689366 | 10118314 | 1.0000 | 0.1211 | 7.24 | 7.34E-05 | INTERGENIC | NA |
| 17 | rs17762691 | 10119173 | 1.0000 | 0.125 | 6.83 | 9.67E-05 | INTERGENIC | NA |
| 17 | exm2272553 | NA | 0.2864 | 0.4727 | 4.75 | 6.60E-05 | NA | NA |
| 19 | rs168109 | 46408111 | 0.5926 | 0.4531 | 3.97 | 8.10E-05 | DOWN-STREAM | NA |

**Table 8: Association Results with $p<10^{-4}$ for Endpoint 1, *PGx-CN-HBePos,* dominant model**

| Chr | SNP | BP | HWE(p) | MAF | Beta | p-value | Variant | Gene |
|---|---|---|---|---|---|---|---|---|
| 3 | rs11923404 | 166614084 | 1.0000 | 0.1614 | 9.83 | 4.25E-05 | INTERGENIC | NA |
| 6 | rs4711668 | 41354451 | 0.4789 | 0.4883 | 0.11 | 6.61E-05 | INTRONIC | TREM1 |
| 6 | rs6899577 | 78017088 | 0.3282 | 0.2344 | 7.17 | 6.99E-05 | INTERGENIC | NA |
| 6 | rs6899965 | 78160992 | 1.0000 | 0.2227 | 6.96 | 8.50E-05 | INTERGENIC | NA |
| 7 | rs16878221 | 14583406 | 0.6738 | 0.3008 | 9.47 | 4.96E-05 | INTRONIC | DGKB |
| 11 | rs2553825 | 35055962 | 0.7952 | 0.2109 | 7.18 | 7.49E-05 | INTERGENIC | NA |
| 17 | rs17689366 | 10118314 | 1.0000 | 0.1211 | 10.78 | 2.11E-05 | INTERGENIC | NA |
| 17 | rs17762691 | 10119173 | 1.0000 | 0.125 | 9.87 | 3.30E-05 | INTERGENIC | NA |
| 19 | rs9630865 | 35471071 | 1.0000 | 0.444 | 0.12 | 8.36E-05 | INTERGENIC | NA |
| 21 | rs3761347 | 33786154 | 0.1143 | 0.2695 | 8.55 | 3.48E-05 | UPSTREAM | NA |
| 21 | rs7283354 | 33798940 | 0.1232 | 0.2812 | 7.26 | 8.94E-05 | INTRONIC | GART |
| 21 | rs7279549 | 33838483 | 0.3892 | 0.2812 | 8.19 | 5.03E-05 | INTRONIC | SON |
| 21 | rs13047599 | 33848130 | 0.5161 | 0.2852 | 8.16 | 5.44E-05 | NON-SYNON | SON |
| 21 | rs12626839 | 33850966 | 0.3771 | 0.2773 | 9.62 | 2.16E-05 | INTRONIC | SON |
| 21 | rs1088256 | 33865413 | 0.3892 | 0.2812 | 8.19 | 5.03E-05 | INTRONIC | SON |
| 21 | rs2834239 | 33876026 | 0.3771 | 0.2773 | 9.59 | 2.25E-05 | INTRONIC | DONSON |
| 21 | rs7283856 | 33881646 | 0.5161 | 0.2852 | 8.13 | 5.66E-05 | INTRONIC | DONSON |
| 21 | rs10460711 | 33885863 | 0.2714 | 0.2734 | 9.65 | 1.99E-05 | INTRONIC | CRYZL1 |
| 21 | rs2070391 | 33949012 | 0.3771 | 0.2773 | 9.93 | 2.04E-05 | INTRONIC | ITSN1 |

(continued)

| Chr | SNP | BP | HWE(p) | MAF | Beta | p-value | Variant | Gene |
|---|---|---|---|---|---|---|---|---|
| 21 | rs2073368 | 34088673 | 0.3960 | 0.293 | 11.73 | 1.34E-05 | INTRONIC | ITSN1 |
| 21 | exm2254590 | NA | 0.6576 | 0.2698 | 8.30 | 5.01E-05 | NA | NA |
| 21 | exm1567802 | NA | 0.3771 | 0.2773 | 9.62 | 2.16E-05 | NA | NA |
| 21 | exm1567815 | NA | 0.5161 | 0.2852 | 8.16 | 5.44E-05 | NA | NA |

**Table 9: Association Results with $p<10^{-4}$ for Endpoint 1, *PGx-GT-HBePos,* dominant model**

| Chr | SNP | BP | HWE (p) | MAF | Beta | p-value | Variant | Gene |
|---|---|---|---|---|---|---|---|---|
| 1 | exm 252809 | NA | 0.726 | 0.4766 | 0.13 | 8.88E-05 | NA | NA |
| 6 | rs4711668 | 41354451 | 0.4789 | 0.4883 | 0.11 | 3.19E-05 | INTRONIC | TREK1 |
| 7 | rs16878221 | 14583406 | 0.6738 | 0.3008 | 7.54 | 7.22E-05 | INTRONIC | DGKB |
| 11 | rs2553825 | 35055962 | 0.7952 | 0.2109 | 7.22 | 5.91E-05 | INTERGENIC | NA |
| 11 | rs2583151 | 99148202 | 0.2742 | 0.0508 | 18.06 | 7.05E-05 | INTERGENIC | NA |
| 17 | rs17689366 | 10118314 | 1.0000 | 0.1211 | 7.65 | 6.53E-05 | INTERGENIC | NA |
| 17 | rs17762691 | 10119173 | 1.0000 | 0.1250 | 7.17 | 9.55E-05 | INTERGENIC | NA |
| 17 | rs1007482 | 69587964 | 1.0000 | 0.1562 | 7.44 | 7.56E-05 | INTERGENIC | NA |
| 19 | rs9630865 | 35471071 | 1.0000 | 0.4440 | 0.11 | 6.72E-05 | INTERGENIC | NA |
| 21 | rs3761347 | 33786154 | 0.1143 | 0.2695 | 7.71 | 3.50E-05 | UPSTREAM | NA |
| 21 | rs7283354 | 33798940 | 0.1232 | 0.2812 | 6.63 | 9.03E-05 | INTRONIC | GART |
| 21 | rs7279549 | 33838483 | 0.3892 | 0.2812 | 7.38 | 5.17E-05 | INTRONIC | SON |
| 21 | rs13047599 | 33848130 | 0.5161 | 0.2852 | 7.35 | 5.61E-05 | NON-SYNON | SON |
| 21 | rs12626839 | 33850966 | 0.3771 | 0.2773 | 8.55 | 2.21E-05 | INTRONIC | SON |
| 21 | rs1088256 | 33865413 | 0.3892 | 0.2812 | 7.38 | 5.17E-05 | INTRONIC | SON |
| 21 | rs2834239 | 33876026 | 0.3771 | 0.2773 | 8.51 | 2.32E-05 | INTRONIC | DONSON |
| 21 | rs7283856 | 33881646 | 0.5161 | 0.2852 | 7.32 | 5.86E-05 | INTRONIC | DONSON |
| 21 | rs 10460711 | 33885863 | 0.2714 | 0.2734 | 8.58 | 2.03E-05 | INTRONIC | CRYZL1 |
| 21 | rs2070391 | 33949012 | 0.3771 | 0.2773 | 8.78 | 2.10E-05 | INTRONIC | ITSN1 |
| 21 | rs2073368 | 34088673 | 0.3960 | 0.2930 | 10.29 | 1.31E-05 | INTRONIC | ITSN1 |
| 21 | exm 254590 | NA | 0.6576 | 0.2698 | 7.42 | 5.25E-05 | NA | NA |
| 21 | exm 567802 | NA | 0.3771 | 0.2773 | 8.55 | 2.21E-05 | NA | NA |
| 21 | exm 567815 | NA | 0.5161 | 0.2852 | 7.35 | 5.61E-05 | NA | NA |

**Table 10: Association Results with $p<10^{-4}$ for Endpoint 2, *PGx-CN-HBePos,* additive model**

| Chr | SNP | BP | HWE(p) | MAF | Beta | p-value | Variant | Gene |
|---|---|---|---|---|---|---|---|---|
| 6 | rs2881194 | 167187909 | 0.5610 | 0.3477 | 8.06 | 7.74E-05 | INTRONIC | RPS6KA2 |
| 6 | exm2270542 | NA | 0.5610 | 0.3477 | 8.06 | 7.74E-05 | NA | NA |
| 6 | rs4710123 | NA | 0.4910 | 0.2578 | 8.85 | 7.29E-05 | NA | NA |
| 14 | rs7145788 | 100827034 | 1.0000 | 0.0703 | 33.93 | 6.89E-05 | INTERGENIC | NA |

(continued)

| Chr | SNP | BP | HWE(p) | MAF | Beta | p-value | Variant | Gene |
|---|---|---|---|---|---|---|---|---|
| 14 | rs2401012 | 100830039 | 0.3580 | 0.1055 | 37.31 | 2.66E-05 | INTERGENIC | NA |
| 14 | rs2151762 | 100865108 | 1.0000 | 0.0703 | 33.93 | 6.89E-05 | INTERGENIC | NA |

**Table 11: Association Results with p<$10^{-4}$ for Endpoint 2, *PGx-GTHBePos,* additive model**

| Chr | SNP | BP | HWE(p) | MAF | Beta | p-value | Variant | Gene |
|---|---|---|---|---|---|---|---|---|
| 8 | rs4460346 | 141182898 | 1.0000 | 0.2070 | 8.84 | 4.36E-05 | INTRONIC | ENSG00000167632 |
| 8 | rs4297022 | 141182923 | 0.7816 | 0.1992 | 9.45 | 4.49E-05 | INTRONIC | ENSG00000167632 |
| 9 | rs17793551 | 111187280 | 0.3662 | 0.1133 | 10.85 | 8.61E-05 | INTRONIC | PTPN3 |
| 11 | rs4936746 | 122172000 | 0.5975 | 0.0859 | 14.18 | 6.26E-05 | INTRONIC | ENSG00000154127 |
| 11 | rs12576789 | 122180319 | 0.5975 | 0.0859 | 14.18 | 6.26E-05 | INTRONIC | ENSG00000154127 |

**Table 12: Association Results with p<$10^{-4}$ for Endpoint 2, *PGx-CN-HBePos,* dominant model**

| Chr | SNP | BP | HWE(p) | MAF | Beta | p-value | Variant | Gene |
|---|---|---|---|---|---|---|---|---|
| 4 | rs13115100 | 160972974 | 0.4772 | 0.1484 | 12.78 | 8.23E-05 | INTERGENIC | NA |
| 11 | rs1320042 | 7400603 | 0.8608 | 0.4883 | 0.04 | 9.86E-05 | INTRONIC | SYT9 |
| 12 | rs2638398 | 19906509 | 0.2807 | 0.4375 | 0.03 | 8.73E-05 | INTERGENIC | NA |
| 12 | rs7959247 | 19909489 | 0.2807 | 0.4375 | 0.03 | 8.73E-05 | INTERGENIC | NA |
| 14 | rs7145788 | 100827034 | 1.0000 | 0.0703 | 33.93 | 6.89E-05 | INTERGENIC | NA |
| 14 | rs2401012 | 100830039 | 0.3580 | 0.1055 | 37.31 | 2.66E-05 | INTERGENIC | NA |
| 14 | rs2151762 | 100865108 | 1.0000 | 0.0703 | 33.93 | 6.89E-05 | INTERGENIC | NA |

**Table 13: Association Results with p<$10^{-4}$ for Endpoint 2, *PGx-GT-HBePos,* dominant model**

| Chr | SNP | BP | HWE(p) | MAF | Beta | p-value | Variant | Gene |
|---|---|---|---|---|---|---|---|---|
| 7 | rs10282247 | 25045068 | 0.1924 | 0.0820 | 19.52 | 5.00E-05 | INTERGENIC | NA |
| 8 | rs4460346 | 141182898 | 1.0000 | 0.2070 | 19.28 | 8.03E-05 | INTRONIC | ENSG00000167632 |
| 8 | rs4297022 | 141182923 | 0.7816 | 0.1992 | 23.66 | 4.42E-05 | INTRONIC | ENSG00000167632 |
| 10 | rs11239257 | 44649855 | 1.0000 | 0.1094 | 13.57 | 9.81E-05 | INTERGENIC | NA |
| 11 | rs1320042 | 7400603 | 0.8608 | 0.4883 | 0.04 | 5.59E-05 | INTRONIC | SYT9 |
| 11 | rs4936746 | 122172000 | 0.5975 | 0.0859 | 14.18 | 6.26E-05 | INTRONIC | ENSG00000154127 |

(continued)

| Chr | SNP | BP | HWE(p) | MAF | Beta | p-value | Variant | Gene |
|---|---|---|---|---|---|---|---|---|
| 11 | rs12576789 | 122180319 | 0.5975 | 0.0859 | 14.18 | 6.26E-05 | INTRONIC | ENSG00000154127 |
| 14 | rs2401012 | 100830039 | 0.3580 | 0.1055 | 16.48 | 6.76E-05 | INTERGENIC | NA |

**Table 14: Association Results with $p<10^{-4}$ for Endpoint 4, *PGx-CN,* additive model**

| Chr | SNP | BP | HWE(p) | MAF | Beta | p-value | Variant | Gene |
|---|---|---|---|---|---|---|---|---|
| 3 | rs1913228 | 62748219 | 1.0000 | 0.1772 | 9.33 | 6.18E-05 | INTRONIC | CADPS |
| 3 | rs12636581 | 62748900 | 1.0000 | 0.1758 | 9.36 | 5.94E-05 | INTRONIC | CADPS |
| 4 | rs10016726 | 14342870 | 0.3099 | 0.3164 | 9.12 | 5.44E-05 | INTERGENIC | NA |
| 6 | exm-rs3830076 | NA | 1.0000 | 0.0820 | 15.77 | 9.64E-05 | NA | NA |
| 8 | rs12675119 | 27714410 | 1.0000 | 0.3398 | 7.63 | 6.20E-05 | INTRONIC | ESCO2 |
| 8 | rs4732756 | 27720605 | 0.6827 | 0.3164 | 8.49 | 2.31E-05 | INTRONIC | ESCO2 |

**Table 15: Association Results with $p<10^{-4}$ for Endpoint 4, *PGx-GT,* additive model**

| Chr | SNP | BP | HWE(p) | MAF | Beta | p-value | Variant | Gene |
|---|---|---|---|---|---|---|---|---|
| 3 | rs7633796 | 62733450 | 0.4034 | 0.1953 | 6.99 | 7.32E-05 | INTRONIC | CADPS |
| 3 | rs1913228 | 62748219 | 1.0000 | 0.1772 | 8.70 | 3.50E-05 | INTRONIC | CADPS |
| 3 | rs12636581 | 62748900 | 1.0000 | 0.1758 | 8.74 | 3.31E-05 | INTRONIC | CADPS |
| 4 | rs6856070 | 10154866 | 1.0000 | 0.1484 | 7.35 | 8.77E-05 | INTRONIC | ENSG00000109684 |
| 4 | rs13108803 | 174915773 | 0.0121 | 0.2305 | 4.91 | 8.15E-05 | INTERGENIC | NA |
| 5 | rs6449558 | 61121309 | 0.1914 | 0.2188 | 5.97 | 9.07E-05 | INTERGENIC | NA |
| 11 | rs10839791 | 7461909 | 0.3640 | 0.1719 | 9.45 | 6.79E-05 | UPSTREAM | NA |
| 11 | rs1038167 | NA | 0.5677 | 0.3633 | 6.14 | 4.38E-05 | NA | NA |
| 12 | rs893531 | 3947012 | 0.3161 | 0.1602 | 9.23 | 8.76E-05 | INTERGENIC | NA |

**Table 16: Association Results with $p<10^{-4}$ for Endpoint 4, *PGx-CN,* dominant model**

| Chr | SNP | BP | HWE(p) | MAF | Beta | p-value | Variant | Gene |
|---|---|---|---|---|---|---|---|---|
| 3 | rs1913228 | 62748219 | 1.0000 | 0.1772 | 18.65 | 2.54E-05 | INTRONIC | CADPS |
| 3 | rs12636581 | 62748900 | 1.0000 | 0.1758 | 18.72 | 2.45E-05 | INTRONIC | CADPS |
| 6 | exm-rs3830076 | NA | 1.0000 | 0.082 | 15.77 | 9.64E-05 | NA | NA |

(continued)

| Chr | SNP | BP | HWE(p) | MAF | Beta | p-value | Variant | Gene |
|---|---|---|---|---|---|---|---|---|
| 8 | rs1437253 | 108864282 | 0.8596 | 0.4922 | 0.07 | 9.40E-05 | INTERGENIC | NA |

**Table 17: Association Results with p<$10^{-4}$ for Endpoint 4, *PGx-GT,* dominant model**

| Chr | SNP | BP | HWE(p) | MAF | Beta | p-value | Variant | Gene |
|---|---|---|---|---|---|---|---|---|
| 3 | rs7633796 | 62733450 | 0.4034 | 0.1953 | 12.02 | 3.88E-05 | INTRONIC | CADPS |
| 3 | rs1913228 | 62748219 | 1.0000 | 0.1772 | 20.06 | 9.27E-06 | INTRONIC | CADPS |
| 3 | rs1913228 | 62748219 | 1.0000 | 0.1772 | 20.06 | 9.27E-06 | INTRONIC | CADPS |
| 3 | rs12636581 | 62748900 | 1.0000 | 0.1758 | 20.16 | 8.81E-06 | INTRONIC | CADPS |
| 3 | rs12636581 | 62748900 | 1.0000 | 0.1758 | 20.16 | 8.81E-06 | INTRONIC | CADPS |
| 3 | rs1513143 | 62753782 | 0.2315 | 0.2422 | 12.11 | 5.59E-05 | INTRONIC | CADPS |
| 4 | rs10014387 | 172643415 | 1.0000 | 0.4961 | 0.10 | 6.92E-05 | INTERGENIC | NA |
| 5 | rs6449558 | 61121309 | 0.1914 | 0.2188 | 12.50 | 6.66E-05 | INTERGENIC | NA |
| 6 | rs1233710 | 28323425 | 0.8444 | 0.3438 | 0.072 | 7.60E-05 | INTRONIC | ZKSCAN4 |
| 6 | rs12000 | 28335415 | 0.5939 | 0.4297 | 0.088 | 8.80E-05 | NON-SYNON | NKAPL |
| 6 | rs2228628 | NA | 1.0000 | 0.1289 | 11.87 | 5.73E-05 | NA | NA |
| 6 | exm524729 | NA | 0.5939 | 0.4297 | 0.088 | 8.80E-05 | NA | NA |
| 6 | rs4713506 | NA | 0.1689 | 0.1523 | 11.54 | 9.28E-05 | NA | NA |
| 6 | exm-rs4713506 | NA | 0.1689 | 0.1523 | 11.54 | 9.28E-05 | NA | NA |
| 6 | rs4713505 | NA | 1.0000 | 0.1289 | 11.87 | 5.73E-05 | NA | NA |
| 6 | exm-rs4713505 | NA | 1.0000 | 0.1289 | 11.87 | 5.73E-05 | NA | NA |

Interpretation

Genome-wide association scanning was applied to 571832 markers with estimated frequency in *PGx-CN* (n=128) of greater than 5%.

Four associations surpassed the level of p<$10^{-5}$ :

- Intronic markers in CADPS (Endpoint 4; PGx-GT; dominant)

CADPS is Calcium-Dependent Secretion Activator. This gene encodes a neural/ endocrine-specific cytosolic and peripheral membrane protein required for the calcium-regulated exocytosis of secretory vesicles. Diseases associated with CADPS include pineoblastoma and childhood medulloblastoma.

Two non-synonymous changes had $p<10^{-4}$:

- rs13047599 in SON (SON DNA Binding Protein)
- rs12000 in NKAPL (NFKB Activating Protein-Like)

The first of these was observed for Endpoint 1, in both *PGx-CN-HBePos* and *PGx-GT-HBePos,* under the dominant model of inheritance, but not the additive model. The encoded protein binds RNA and promotes pre-mRNA splicing, particularly transcripts with poor splice sites. The protein also recognizes a specific DNA sequence found in the human hepatitis B virus (HBV) and represses HBV core promoter activity. Diseases associated with SON include hepatitis B. Bar plots, showing rate of response by genotype under the dominant model, for rs 13047599 and Endpoint 1 is given in Figure 9 below.

The second non-synonymous change was observed for Endpoint 4 in *PGx-GT.* It lies in NKAPL, a protein-coding gene associated with schizophrenia. Bar plots, showing rate of response by genotype for rs12000 and Endpoint 4 is given in Figure 10 below.

As expected, a great deal of consistency of results was observed within each endpoint; the difference between the *CN* and *GT* groups is a matter of only a handful of patients. A total of 33 genes are listed in the tables above. Of these, at least two have been previously implicated in hepatitis B disease risk or progression:

- PTPN3 (Protein tyrosine phosphatase; Hsu et al, 2007)
- TREM1 (Triggering receptor expressed on myeloid cells 1; Liao et al, 2012)

<u>Software</u>

Custom-written perl scripts (Wall et al, 1996) were used to reformat the data, select markers for ancestry analysis and produce tables. PLINK version 1.07 (Purcell et al, 2007) was used to perform the genetic QC analyses, to merge study data with HapMap data, and for association analysis. EIGENSOFT 4.0 (Patterson et al, 2006; Price et al, 2006) was used for PCA. R version 2.15.2 (R Core Team, 2012) was used for the production of graphics.

## Claims

1. A method of identifying a patient who may benefit from treatment with an anti-HBV therapy comprising an interferon, the method comprising:

   determining the presence of a single nucleotide polymorphism in gene *SON* on chromosome 21 in a sample obtained from the patient, wherein the presence of at least one G allele at rs13047599 indicates that the patient may benefit from the treatment with the anti-HBV treatment.

2. A method for predicting HBeAg seroconversion and HBV DNA <2000 IU/ml at >=24-week follow-up of treatment of an HBe-positive patient infected with HBV to interferon treatment comprising: detecting the presence of a single nucleotide polymorphism in gene *SON* on chromosome 21 in a sample obtained from said patient, and determining that said patient has a high response rate to interferon treatment measured as HBeAg seroconversion and HBV DNA <2000 IU/ml at >=24-week follow-up of treatment if at least one G allele at rs13047599 is present.

3. The method of any of claims 1 or 2, wherein the interferon is selected from the group of peginterferon alfa-2a, peginterferon alfa-2b, interferon alfa-2a and interferon alfa-2b.

4. The method of claim 3, wherein the interferon is a peginterferon alfa-2a conjugate having the formula:

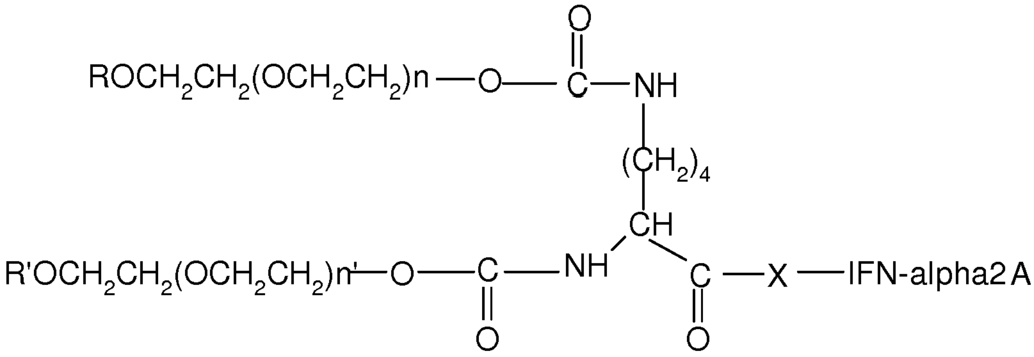

wherein R and R' are methyl, X is NH, and n and n' are individually or both either 420 or 520.

## Patentansprüche

**1.** Verfahren zum Identifizieren eines Patienten, der von einer Behandlung mit einer Anti-HBV-Therapie profitieren kann, die ein Interferon umfasst, wobei das Verfahren umfasst:

Ermitteln des Vorhandenseins eines Einzelnukleotid-Polymorphismus im Gen *SON* auf Chromosom 21 in einer vom Patienten erhaltenen Probe, wobei das Vorhandensein mindestens eines G-Allels bei rs13047599 anzeigt, dass der Patient von der Behandlung mit der Anti-HBV-Behandlung profitieren kann.

**2.** Verfahren zur Vorhersage von HBeAg-Serokonversion und HBV-DNA <2000 IE/ml bei > = 24-wöchiger Beobachtung nach der Behandlung des HBe- positiven Patienten, der mit HBV infiziert ist, zur Interferonbehandlung, umfassend:

Nachweisen des Vorhandenseins eines einzelnen Nukleotidpolymorphismus im Gen *SON* auf Chromosom 21 in einer Probe, die vom Patienten erhalten wurde, und Feststellen, dass der Patient eine hohe Ansprechrate auf Interferonbehandlung aufweist, gemessen als HBeAg- Serokonversion und HBV-DNA <2000 IE/ml bei > = 24-wöchiger Beobachtung nach der Behandlung, wenn mindestens ein G-Allel bei rs13047599 vorhanden ist.

**3.** Verfahren nach einem der Ansprüche 1 oder 2, wobei das Interferon ausgewählt ist aus der Gruppe von Peginterferon alfa-2a, Peginterferon alfa-2b, Interferon alfa-2a und Interferon alfa-2b.

**4.** Verfahren nach Anspruch 3, wobei das Interferon ein Peginterferon-alfa-2a-Konjugat ist mit der Formel:

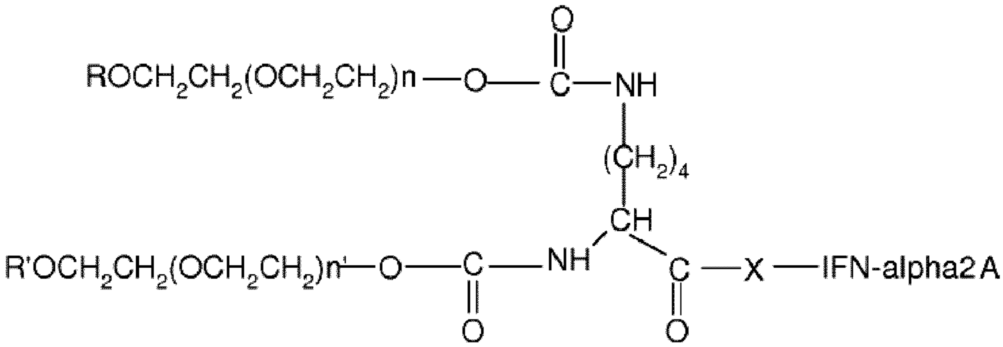

wobei R und R' Methyl sind, X NH ist und n und n' einzeln oder beide entweder 420 oder 520 sind.

## Revendications

**1.** Procédé d'identification d'un patient pouvant bénéficier d'un traitement avec une thérapie contre le virus de l'hépatite B comprenant un interféron, le procédé consistant à :

déterminer la présence d'un polymorphisme nucléotidique simple dans le gène *SON* sur le chromosome 21 dans un échantillon prélevé sur le patient, la présence d'au moins un allèle G au niveau de rs13047599 indiquant que le patient peut bénéficier du traitement avec la thérapie contre le virus de l'hépatite B.

**2.** Procédé permettant de prédire une séroconversion HBeAg et l'ADN du virus de l'hépatite B <2000 UI/ml à un suivi de traitement >= 24 semaines d'un patient HBe-positif infecté par le virus de l'hépatite B au traitement par interféron consistant à :

détecter la présence d'un polymorphisme nucléotidique simple dans le gène *SON* sur le chromosome 21 dans un échantillon prélevé sur ledit patient, et à déterminer que ledit patient présente un taux de réponse élevé au traitement par interféron mesuré en tant que séroconversion HBeAg et un ADN du virus de l'hépatite B <2000 UI/ml à un suivi de traitement >= 24 semaines si au moins un allèle G en rs13047599 est présent.

**3.** Procédé selon l'une quelconque des revendications 1 ou 2, dans lequel l'interféron est choisi dans le groupe comprenant l'interféron pégylé alfa-2a, l'interféron pégylé alfa-2b, l'interféron alfa-2a et l'interféron alfa-2b.

**4.** Procédé selon la revendication 3, dans lequel l'interféron est un conjugué d'interféron pégylé alfa-2a de formule :

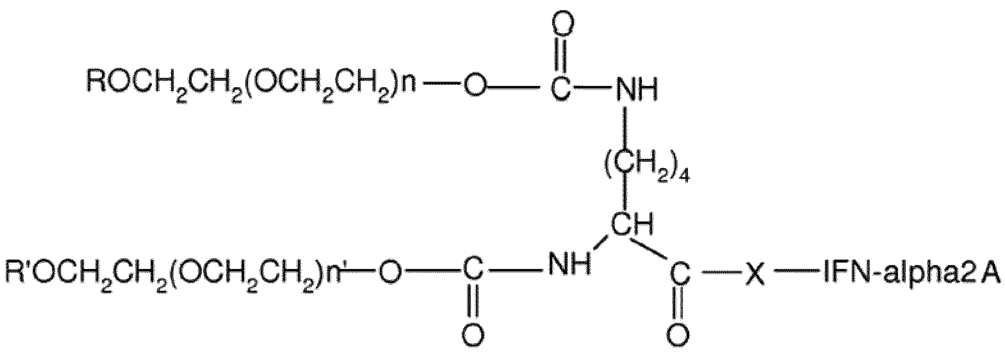

dans laquelle R et R' sont le méthyle, X est NH, et n et n' valent individuellement ou tous les deux 420 ou 520.

Fig. 1

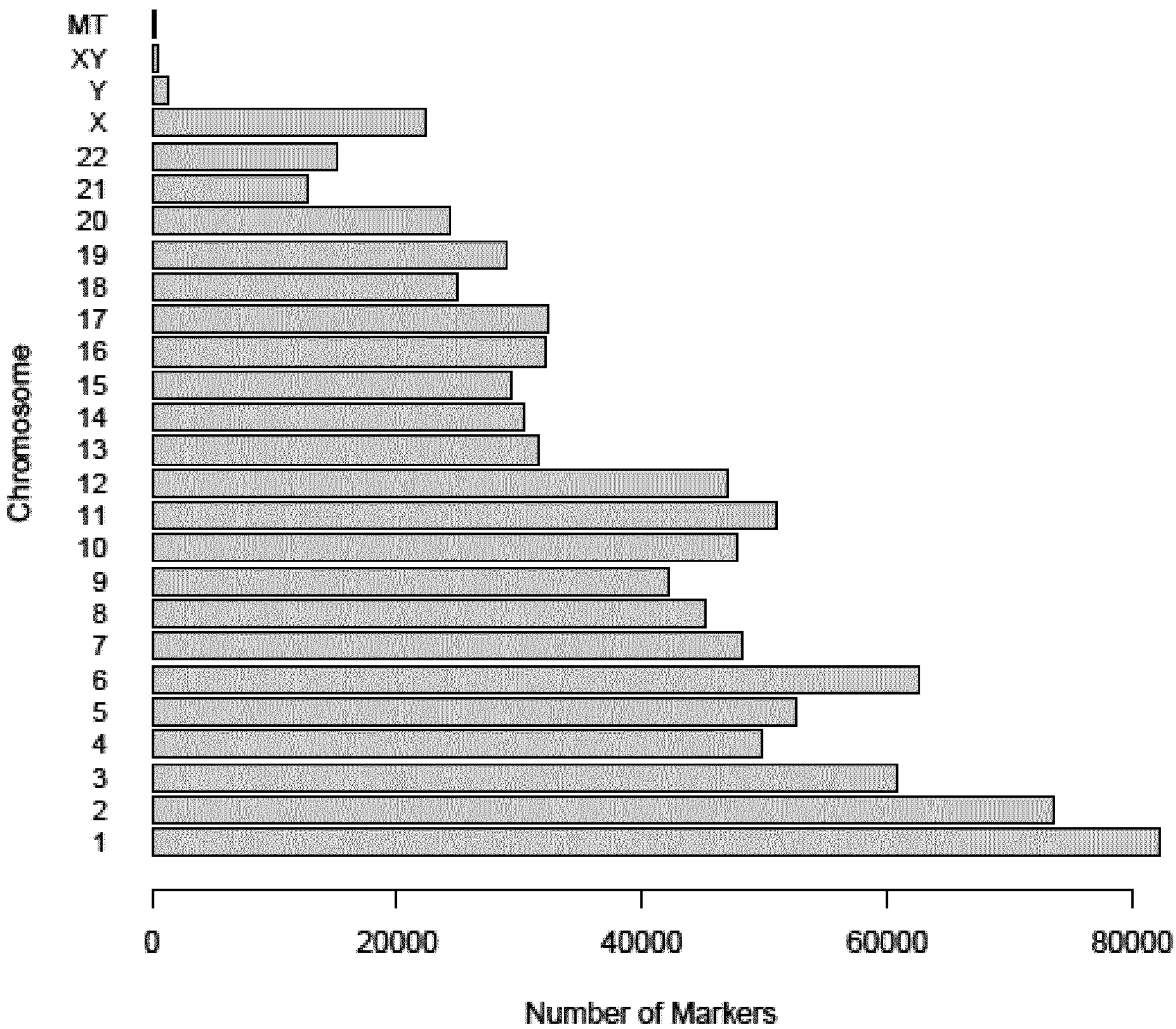
MT
XY
Y
X
22
21
20
19
18
17
16
15
14
13
12
11
10
9
8
7
6
5
4
3
2
1
Chromosome
0
20000
40000
60000
80000
Number of Markers

Fig. 2

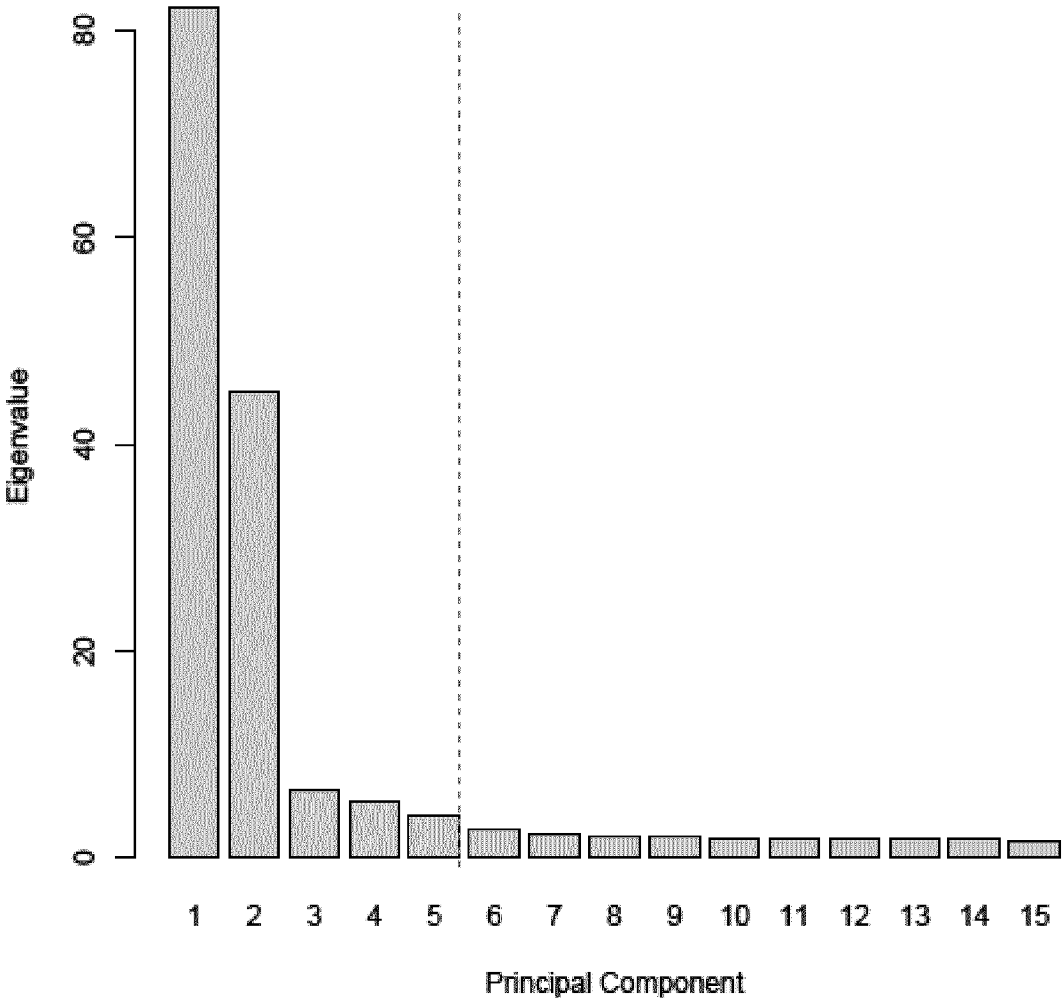
80
60
40
20
0
Eigenvalue
1
2
3
4
5
6
7
8
9
10
11
12
13
14
15
Principal Component

Fig. 3

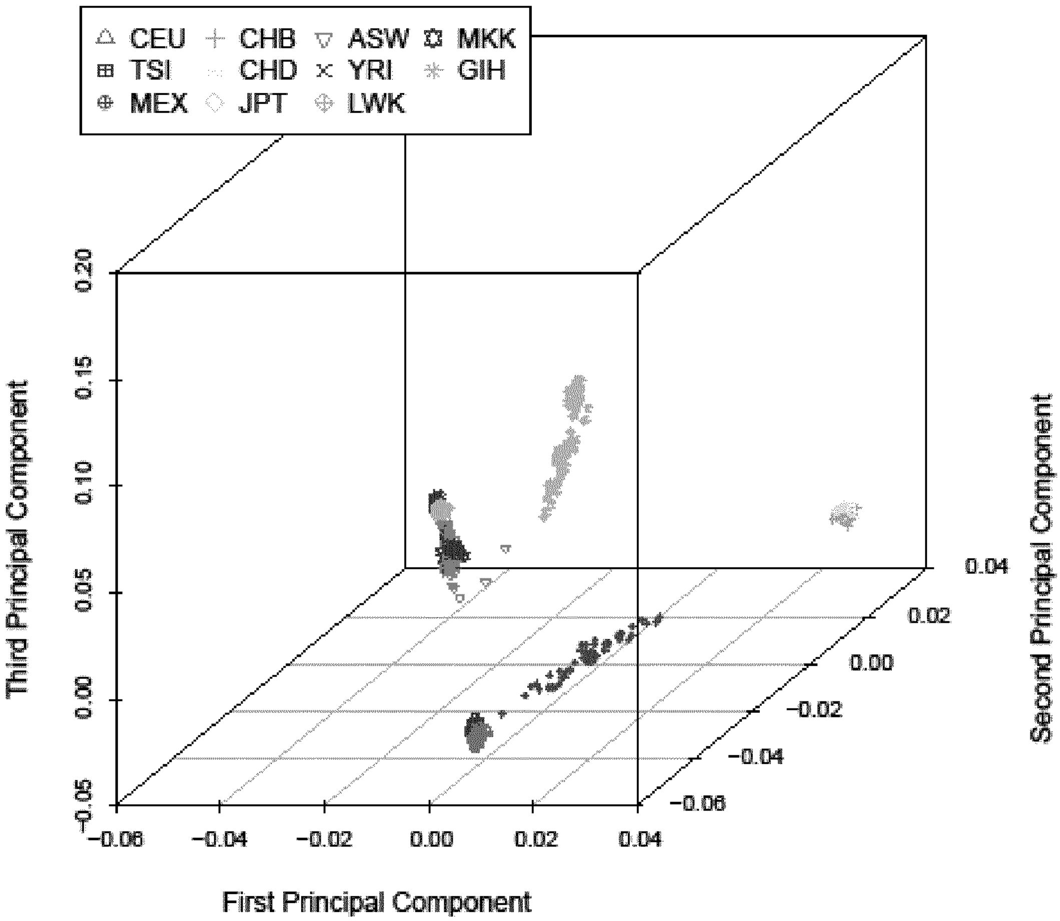
CEU
CHB
ASW
MKK
TSI
CHD
YRI
GIH
MEX
JPT
LWK
Third Principal Component
0.20
0.15
0.10
0.05
0.00
−0.05
−0.06
−0.04
−0.02
0.00
0.02
0.04
First Principal Component
Second Principal Component
0.04
0.02
0.00
−0.02
−0.04
−0.06

Fig. 4

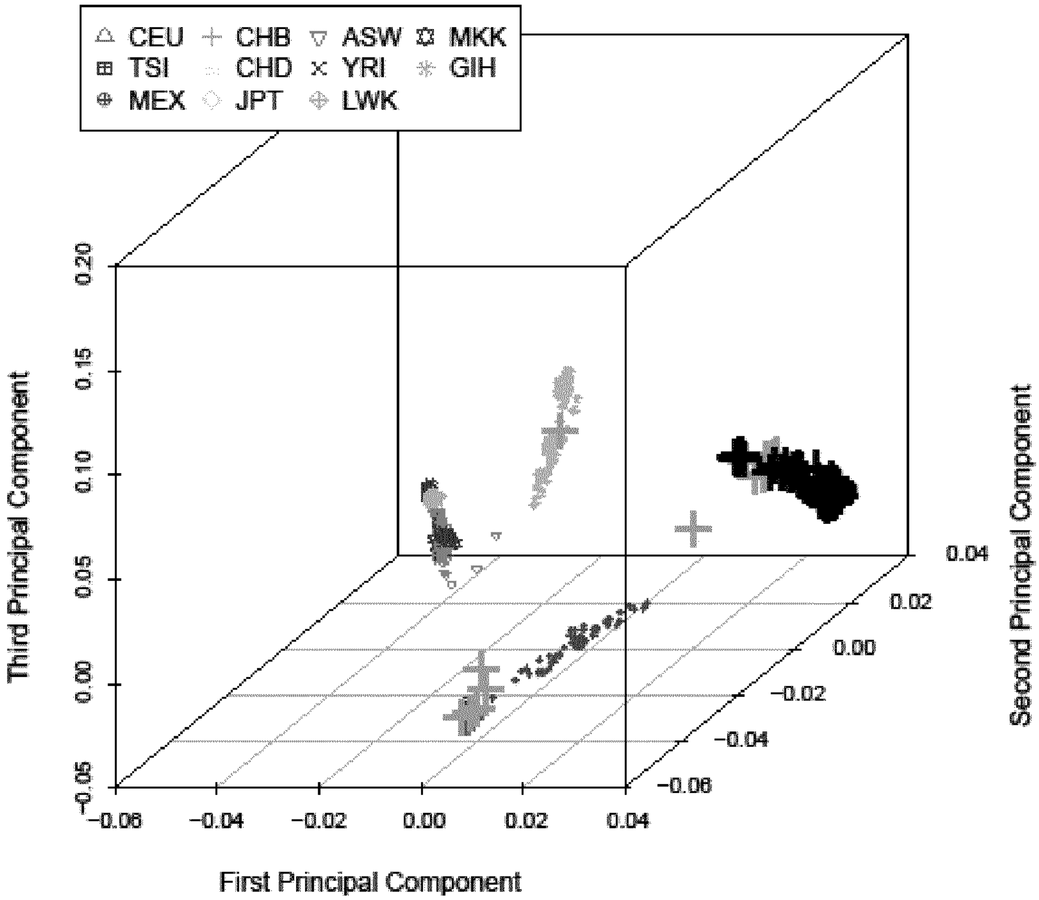
CEU
CHB
ASW
MKK
TSI
CHD
YRI
GIH
MEX
JPT
LWK
Third Principal Component
Second Principal Component
First Principal Component
-0.05
0.00
0.05
0.10
0.15
0.20
-0.06
-0.04
-0.02
0.00
0.02
0.04

Fig. 5

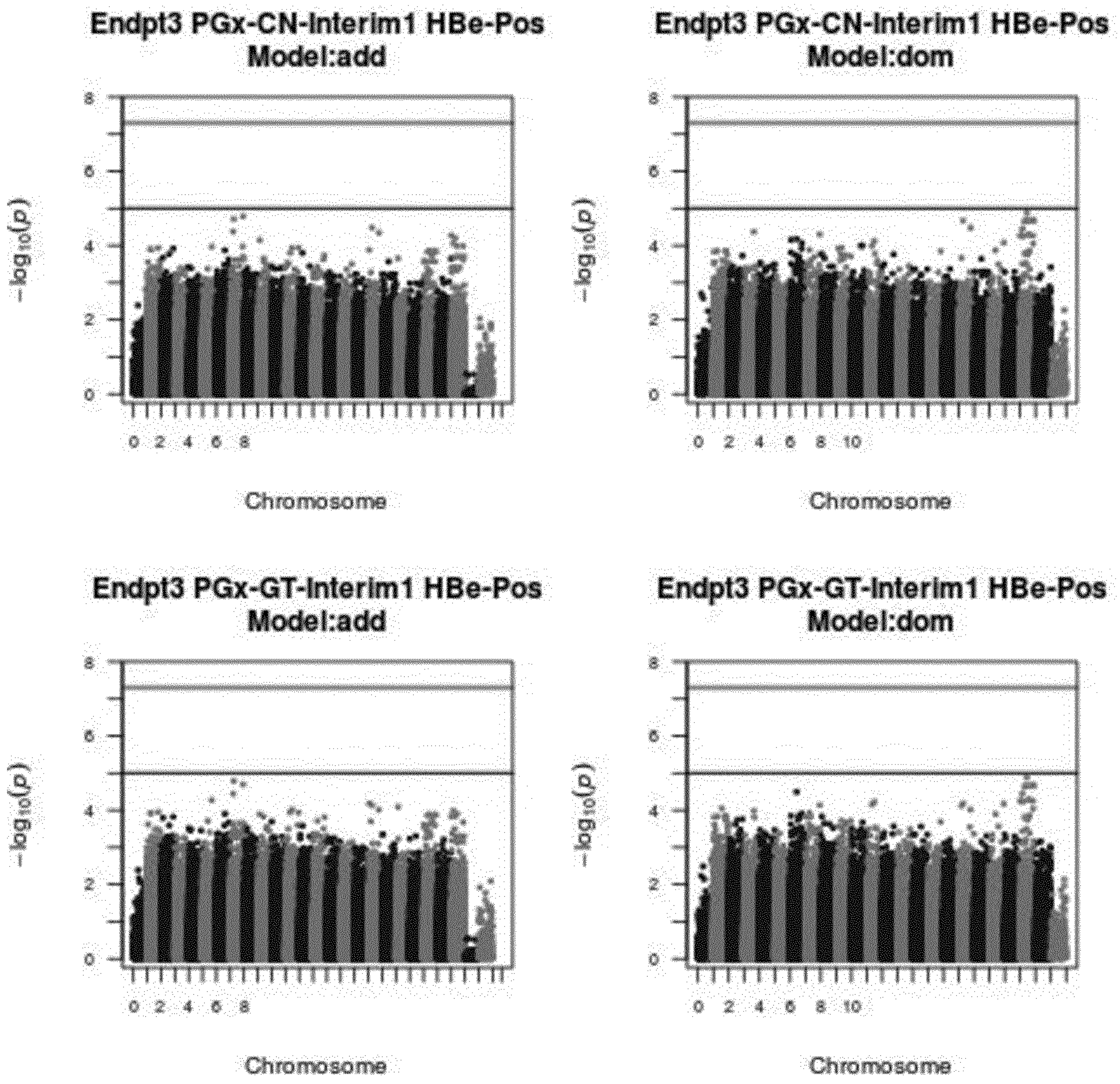
Endpt3 PGx-CN-Interim1 HBe-Pos
Model:add
Endpt3 PGx-CN-Interim1 HBe-Pos
Model:dom
Endpt3 PGx-GT-Interim1 HBe-Pos
Model:add
Endpt3 PGx-GT-Interim1 HBe-Pos
Model:dom
$-\log_{10}(p)$
0 2 4 6 8
0 2 4 6 8 10
Chromosome

Fig. 6

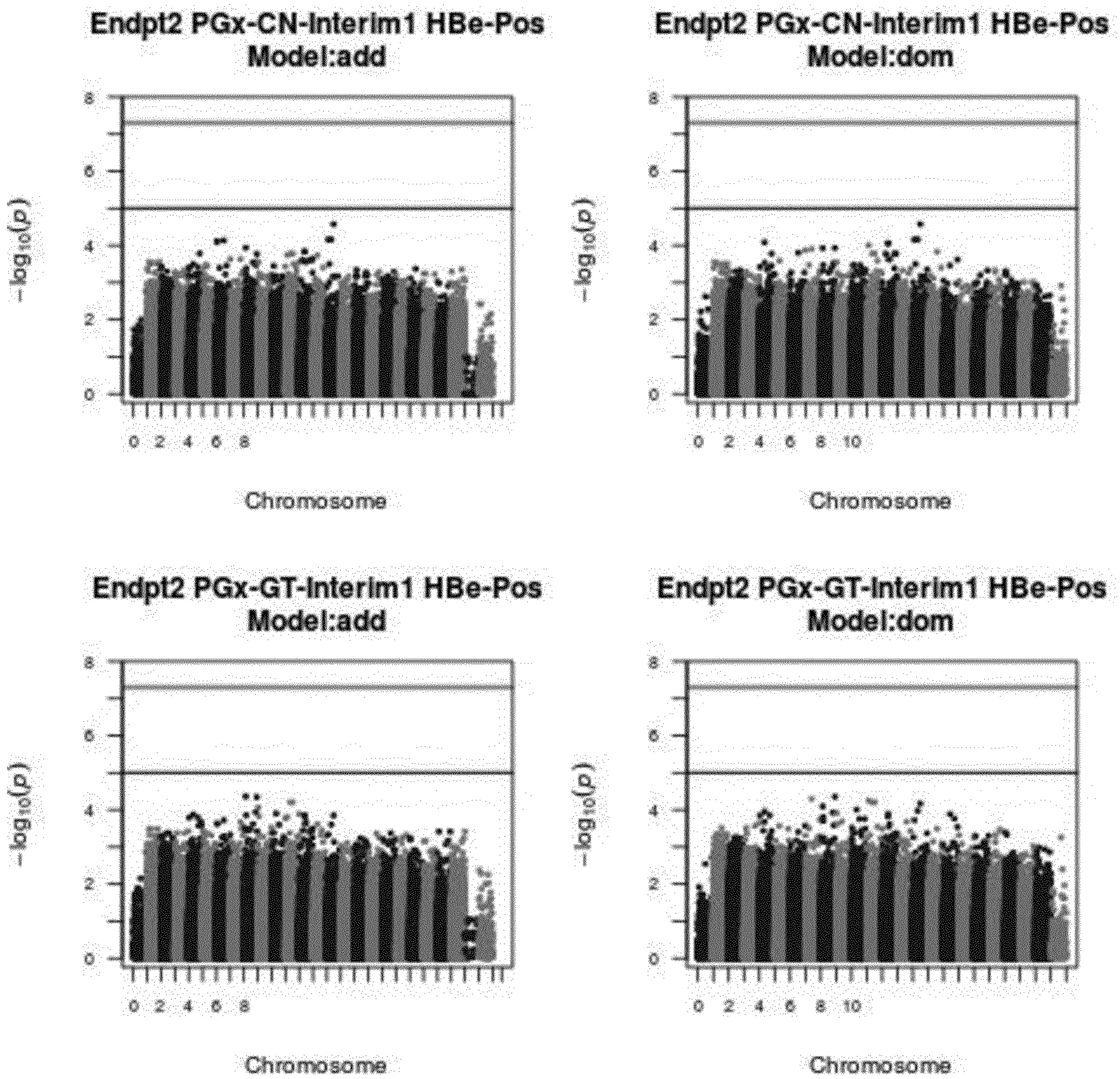
Endpt2 PGx-CN-Interim1 HBe-Pos
Model:add
−log10(p)
8
6
4
2
0
0 2 4 6 8
Chromosome
Endpt2 PGx-CN-Interim1 HBe-Pos
Model:dom
−log10(p)
8
6
4
2
0
0 2 4 6 8 10
Chromosome
Endpt2 PGx-GT-Interim1 HBe-Pos
Model:add
−log10(p)
8
6
4
2
0
0 2 4 6 8
Chromosome
Endpt2 PGx-GT-Interim1 HBe-Pos
Model:dom
−log10(p)
8
6
4
2
0
0 2 4 6 8 10
Chromosome

Fig. 7

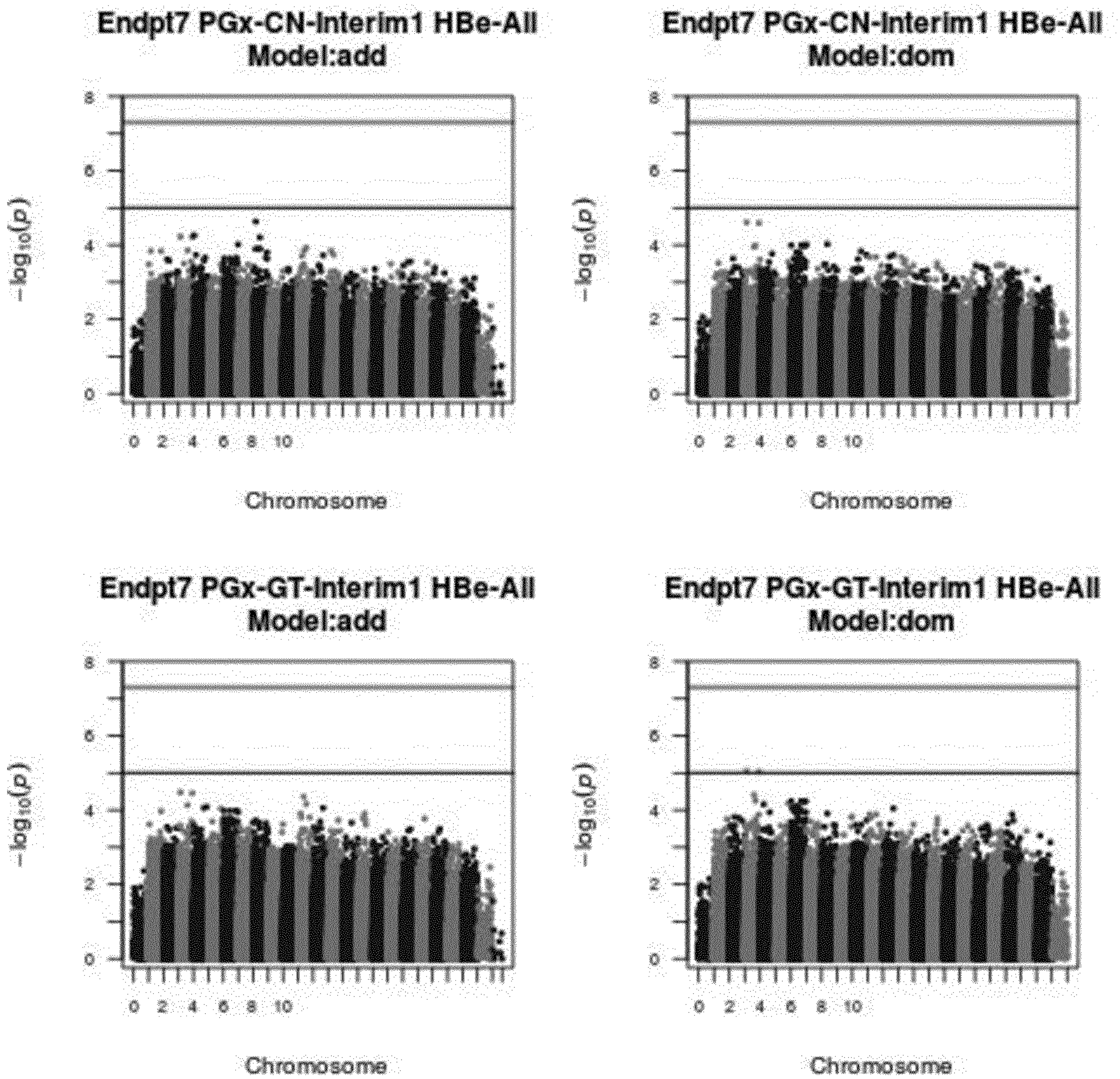
Endpt7 PGx-CN-Interim1 HBe-All
Model:add
Endpt7 PGx-CN-Interim1 HBe-All
Model:dom
Endpt7 PGx-GT-Interim1 HBe-All
Model:add
Endpt7 PGx-GT-Interim1 HBe-All
Model:dom
−log10(p)
Chromosome
0 2 4 6 8 10
0 2 4 6 8

Fig. 8

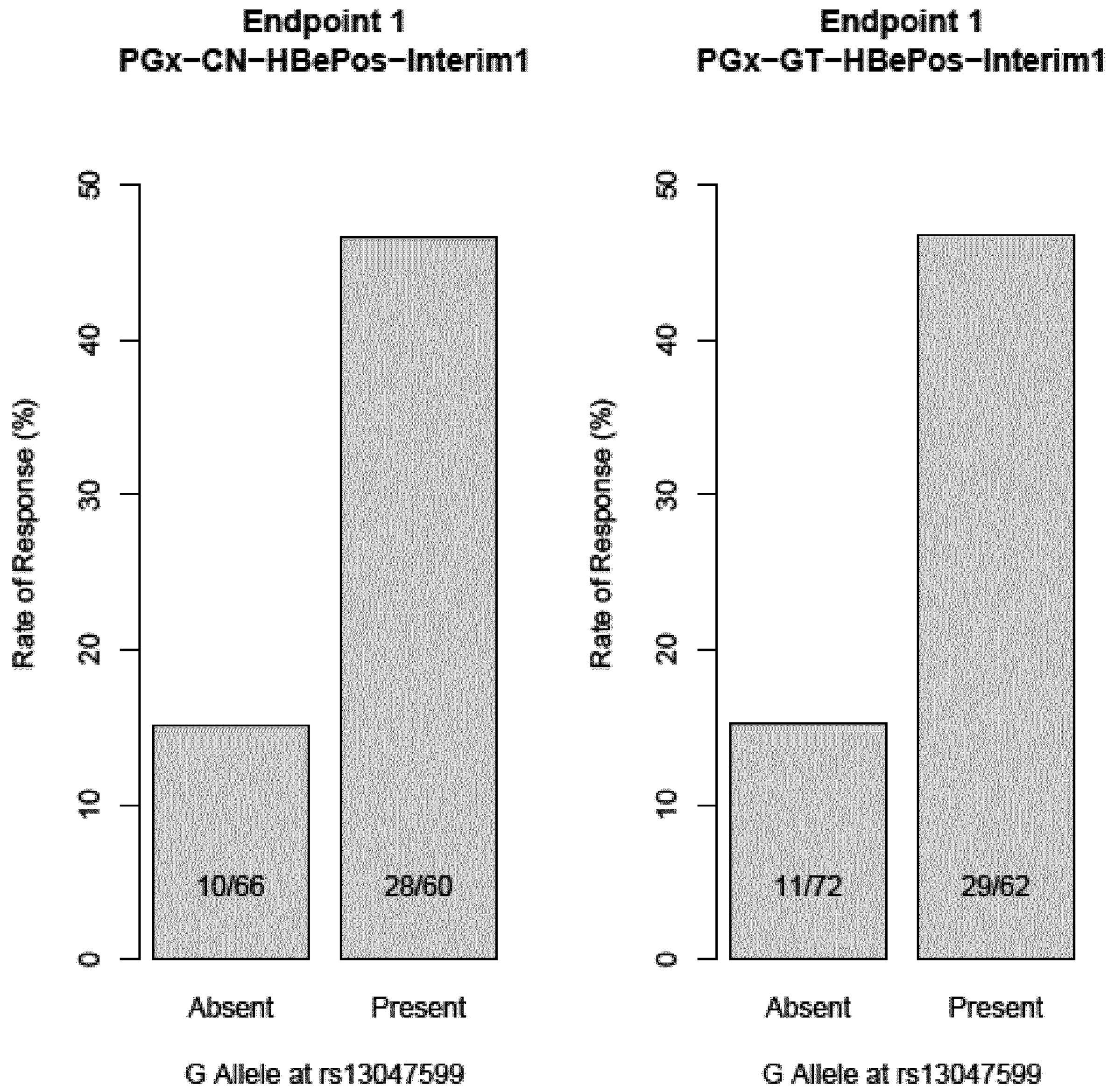
Endpoint 1
PGx−CN−HBePos−Interim1
Rate of Response (%)
0
10
20
30
40
50
10/66
28/60
Absent
Present
G Allele at rs13047599
Endpoint 1
PGx−GT−HBePos−Interim1
Rate of Response (%)
0
10
20
30
40
50
11/72
29/62
Absent
Present
G Allele at rs13047599

Fig. 9

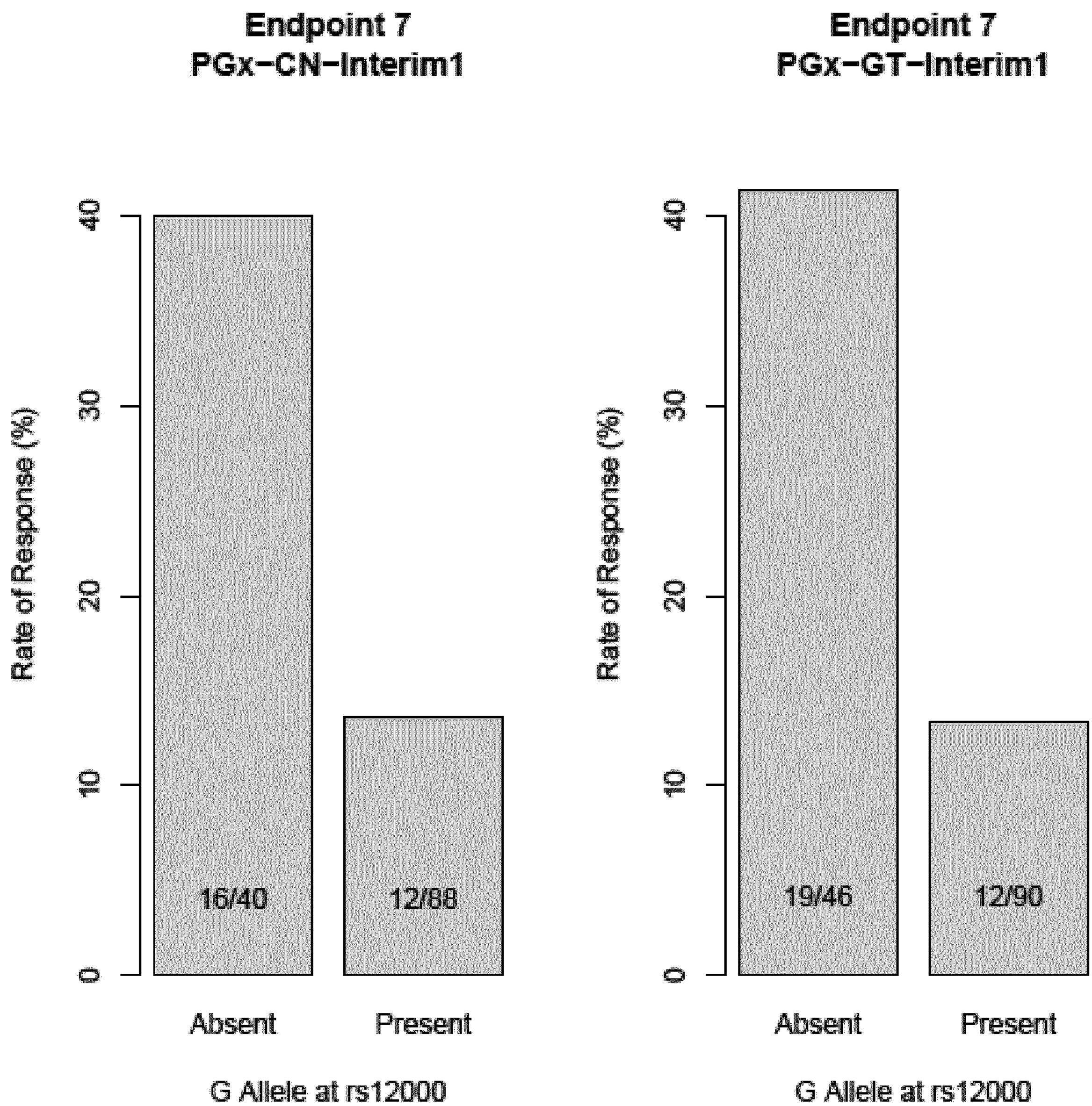
Endpoint 7
PGx−CN−Interim1
Rate of Response (%)
0
10
20
30
40
16/40
12/88
Absent
Present
G Allele at rs12000
Endpoint 7
PGx−GT−Interim1
Rate of Response (%)
0
10
20
30
40
19/46
12/90
Absent
Present
G Allele at rs12000

Fig. 10

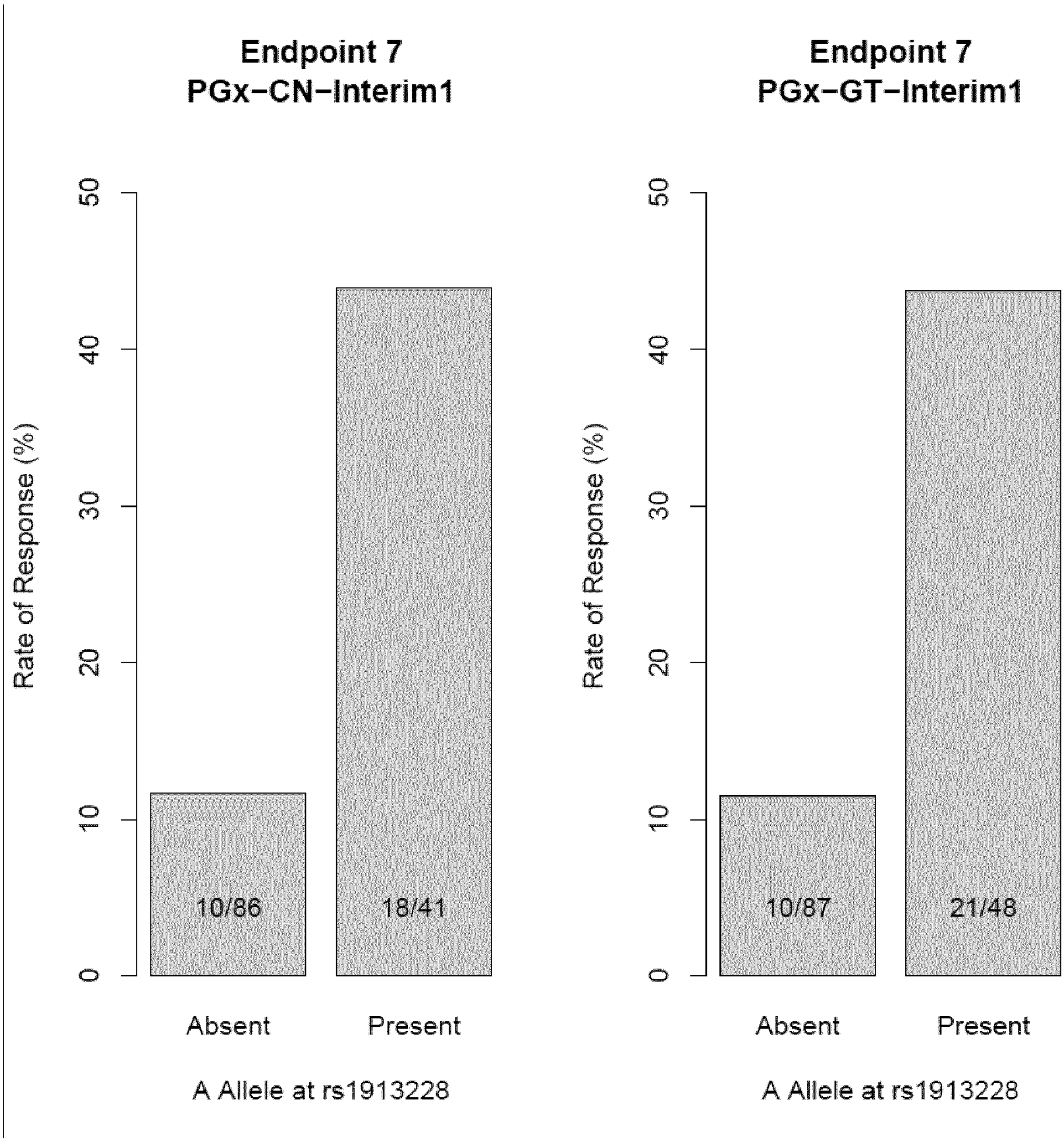
Endpoint 7
PGx−CN−Interim1
Rate of Response (%)
0
10
20
30
40
50
10/86
18/41
Absent
Present
A Allele at rs1913228
Endpoint 7
PGx−GT−Interim1
Rate of Response (%)
0
10
20
30
40
50
10/87
21/48
Absent
Present
A Allele at rs1913228

Fig. 11

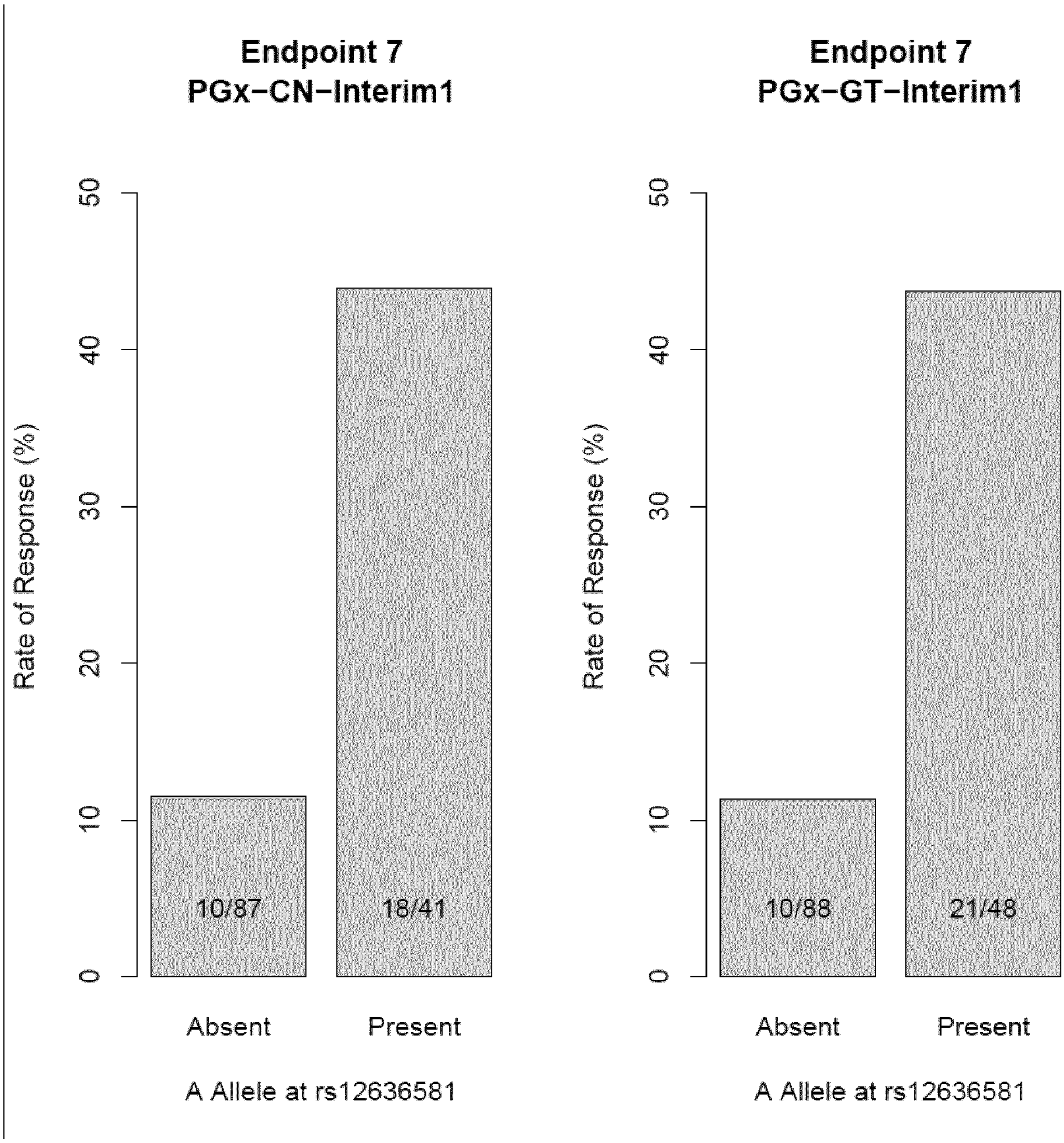
Endpoint 7
PGx−CN−Interim1
Rate of Response (%)
0
10
20
30
40
50
10/87
18/41
Absent
Present
A Allele at rs12636581
Endpoint 7
PGx−GT−Interim1
Rate of Response (%)
0
10
20
30
40
50
10/88
21/48
Absent
Present
A Allele at rs12636581

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4897471 A
- US 4695623 A


### Non-patent literature cited in the description


- **DIENSTAG J.L. ; HEPATITIS B.** *Virus Infection., N. Engl. J. Med.,* 2008, vol. 359, 1486-1500
- **MULLER U. ; STEINHOFF U. ; REIS L.F. et al.** Functional role of type I and type II interferons in antiviral defense. *Science,* 1994, vol. 264, 1918-21
- **MARCELLIN P. ; LAU G.K. ; BONINO F. et al.** Peginterferon alfa-2a alone, lamivudine alone, and the two in combination in patients with HBeAg-negative chronic hepatitis B. *N. Engl. J. Med.,* 2004, vol. 351, 1206-17
- **LAU G.K. ; PIRATVISUTH T ; LUO K.X. et al.** Peginterferon Alfa-2a, Lamivudine, and the Combination for HBeAg-Positive Chronic Hepatitis B. *N. Engl. J. Med.,* 2005, vol. 352, 2682-95
- **LI W.C. ; WANG M.R. ; KONG L.B. et al.** Peginterferon alpha-based therapy for chronic hepatitis B focusing on HBsAg clearance or seroconversion: a meta-analysis of controlled clinical trials. *BMC Infect. Dis.,* 2011, vol. 11, 165-177
- **LIAW Y.F. ; JIA J.D. ; CHAN H.L. et al.** Shorter durations and lower doses of peginterferon alfa-2a are associated with inferior hepatitis B e antigen seroconversion rates in hepatitis B virus genotypes B or C. *Hepatology,* 2011, vol. 54, 1591-9
- **KAMATANI Y. ; WATTANAPOKAYAKIT S. ; OCHI H. et al.** A genome-wide association study identifies variants in the HLA-DP locus associated with chronic hepatitis B in Asians. *Nat. Genet.,* 2009, vol. 41, 591-595
- **GUO X. ; ZHANG Y. ; LI J. et al.** Strong influence of human leukocyte antigen (HLA)-DP gene variants on development of persistent chronic hepatitis B virus carriers in the Han Chinese population. *Hepatology,* 2011, vol. 53, 422-8
- **NISHIDA N. ; SAWAI H. ; MATSUURA K. et al.** Genome-wide association study confirming association of HLA-DP with protection against chronic hepatitis B and viral clearance in Japanese and Korean. *PLos One,* 2012, vol. 7, e39175
- **MBAREK H. ; OCHI H. ; URABE Y. et al.** A genome-wide association study of chronic hepatitis B identified novel risk locus in a Japanese population. *Hum. Mol. Genet.,* 2011, vol. 20, 3884-92
- **GE D. ; FELLAY J. ; THOMPSON A.J. et al.** Genetic variation in IL28B predicts hepatitis C treatment-induced viral clearance. *Nature,* 2009, vol. 461, 399-401
- **TANAKA Y. ; NISHIDA N. ; SUGIYAMA M. et al.** Genome-wide association of IL28B with response to pegylated interferon-alpha and ribavirin therapy for chronic hepatitis C. *Nat. Genet.,* 2009, vol. 41, 1105-9
- **SUPPIAH V. ; MOLDOVAN M. ; AHLENSTIEL G. et al.** IL28B is associated with response to chronic hepatitis C interferon-alpha and ribavirin therapy. *Nat. Genet.,* 2009, vol. 41, 1100-4
- **LAMPERTICO P. ; VIGANO M. ; CHERONI C. et al.** Genetic variation in IL28B polymorphism may predict HBsAg clearance in genotype D, HBeAg negative patients treated with interferon alfa. *AASLD,* 2010
- **MANGIA A. ; SANTORO R. ; MOTTOLA et al.** Lack of association between IL28B variants and HBsAg clearance after interferon treatment. *EASL,* 2011
- **DE NIET A. ; TAKKENBERG R.B. ; BENAYED R. et al.** Genetic variation in IL28B and treatment outcome in HBeAg-positive and -negative chronic hepatitis B patients treated with Peg interferon alfa-2a and adefovir. *Scand. J. Gastroenterol.,* 2012, vol. 47, 475-81
- **SONNEVELD M.J. ; WONG V.W. ; WOLTMAN A.M. et al.** Polymorphisms near IL28B and serologic response to peginterferon in HBeAg-positive patients with chronic hepatitis B. *Gastroenterology,* 2012, vol. 142, 513-520
- **HOLMES et al.** IL28B genotype is not useful for predicting treatment outcome in Asian chronic hepatitis B patients treated with pegylated interferon-alpha. *J. Gastroenterol. Hepatol.,* 2013, vol. 28 (5), 861-6